# EUROPÄISCHE PATENTSCHRIFT

(11) **EP 2 414 327 B1**
(45) Veröffentlichungstag und Bekanntmachung des Hinweises auf die Patenterteilung: **19.11.2014**
(21) Anmeldenummer: 10706554.2
(22) Anmeldetag: 03.03.2010
(51) Int. Cl.: C07D 209/08, C07D 231/56, C07D 249/18, C07D 263/58, C07D 401/12, C07D 403/12, C07D 413/06, C07D 413/12, A61K 31/496, A61K 31/4523, A61P 35/00

(54) **HETEROCYCLISCHE VERBINDUNGEN ALS AUTOTAXIN-INHIBITOREN**
HETEROCYCLIC COMPOUNDS AS AUTOTAXIN INHIBITORS
COMPOSÉS HÉTÉROCYCLIQUES COMME INHIBITEURS DE L'AUTOTAXINE

(30) Priorität: 02.04.2009 EP 09004858
(43) Veröffentlichungstag der Anmeldung: 08.02.2012
(73) Patentinhaber: Merck Patent GmbH, 64293 Darmstadt (DE)
(72) Erfinder: STAEHLE, Wolfgang, 55218 Ingelheim (DE); SCHIEMANN, Kai, 64342 Seeheim-Jugenheim (DE); SCHULTZ, Melanie, 64287 Darmstadt (DE)
(86) Internationale Anmeldenummer: PCT/EP2010/001324
(87) Internationale Veröffentlichungsnummer: WO 2010/112116

(56) Entgegenhaltungen:
- WO-A1-02/30422
- WO-A1-02/080928
- WO-A2-2009/046841
- PARRILL ABBY L ET AL: "Virtual screening approaches for the identification of non-lipid autotaxin inhibitors" BIOORGANIC & MEDICINAL CHEMISTRY, Bd. 16, Nr. 4, Februar 2008 (2008-02), Seiten 1784-1795, XP002578964 ISSN: 0968-0896

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen zur Behandlung von Krankheiten, die mit einer Erhöhung des Lysophosphatsäure Spiegels einhergehen, ferner pharmazeutische Zusammensetzungen, die diese Verbindungen enthalten.

Im einzelnen betrifft die vorliegende Erfindung Verbindungen der Formel I, die bevorzugt eines oder mehrere Enzyme hemmen, die den Lysophosphatsäure (*lysophosphatidic acid oder* abgekürzt LPA) Spiegel regulieren und/oder modulieren, Zusammensetzungen, die diese Verbindungen enthalten, sowie Verfahren zu ihrer Verwendung zur Behandlung von Krankheiten und Leiden wie Angiogenese, Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstossung. Insbesondere eignen sich die erfindungsgemäßen Verbindungen zur Therapie oder Prophylaxe von Krebserkrankungen.

Autotaxin (ATX) ist eine Enzym welches für die Erhöhung des Lysophosphatsäurespiegel in Ascites und Plasma verantwortlich ist (Xu et al. 1995, Clinical Cancer Research Vol. 1, Seite 1223 und Xu et al. 1995, Biochem. J. Vol- 309, Seite 933). ATX setzt Lysophatidylcholin (LPC) zu Lysophosphatsäure um (Tokumura et al. 2002, J. Biol. Chem., Vol 277, Seite 39436 und Umezu-Gozo et al. 2002, J. Biol. Chem., Vol. 158, Seite 227) LPA ist ein interzellularer Lipid Mediator der eine Vielzahl von biologischen und biochemischen Prozessen wie beispielsweise glatte Muskelkontraktion, Thrombozyten Aggregation und Apoptose beeinflusst (Tigyi et al. 2003 Prog. Lipid Res. Vol 42 , Seite. 498 und Mills et al. 2003 Nat. Rev. Cancer Vol. 3, Seite 582 und Lynch et al. 2001 Prost. Lipid Med. Vol.64, Seite 33). Außerdem ist LPA in erhöhten Konzentrationen in Plasma und Ascites Flüssigkeit von Ovarial Krebs Patienten der frühen und späten Phase zu finden. LPA spielt dort eine Rolle bei der Tumorzellproliferation und deren Invasion in benachbarte Gewebe, welche zur Metastasierung führen kann (Xu et al. 1995, Clinical Cancer Research Vol. 1, Seite 1223 und Xu et al. 1995, Biochem. J. Vol- 309, Seite 933). Diese biologischen und phatobiologischen Prozesse werden durch die Aktivierung durch LPA von G-Protein gekoppelten Rezeptoren angeschaltet (Contos et al. 2000, Mol. Pharm. Vol 58, Seite. 1188).

Aus diesem Grunde ist es zur Behandlung von Tumorpatienten wünschenswert, den LPA Spiegel zu senken. Dies kann durch die Hemmung von Enzymen erreicht werden, die an der LPA Biosynthese beteiligt sind, wie beispielsweise Autotaxin (ATX, Sano et al. 2002, J. Biol. Chem. Vol. 277 , Seite 21197 und Aoki et al. 2003, J. Biol. Chem. Vol. 277 Seite 48737). Autotaxin gehört zu der Enzym Familie der Nukleotide Pyrophosphatasen und Phosphodiesterasen (Goding et al. 1998, Immunol. Rev. Vol. 161, Seite 11) und stellt einen wichtigen Ansatzpunkt bei der antitumoralen Therapie dar (Mills et al. 2003 Nat. Rev. Cancer Vol. 3, Seite 582 and Goto eta I. 2004 J. Cell. Biochem. Vol. 92, Seite 1115), da es in Tumoren verstärkte expremiert wird und Tumorzellproliferation und -invasion in benachbarte Gewebe, was zur Metastasenbildung führen kann, bewirkt (Nam et al. 2000, Oncogene, Vol. 19 Seite 241). Außerdem bewirkt Autotaxin zusammen mit anderen angiogenetischen Faktoren Blutgefäßformation im Rahmen der Angiogenese (Nam et al. 2001, Cancer Res. Vol. 61 Seite. 6938). Angiogenese ist ein wichtiger Vorgang beim Tumorwachstum, der die Versorgung des Tumors mit Nährstoffen sichert. Aus diesem Grunde ist die Hemmung der Angiogenese ein wichtiger Ansatzpunkt der Krebs- und Tumortherapie, mit dem der Tumor gewissermaßen ausgehungert werden kann (Folkman, 2007, Nature Reviews Drug Discovery Vol. 6, Seite 273-286).

Es wurde überraschend gefunden, dass die erfindungsgemäßen Verbindungen eine spezifische Inhibierung der Enzymfamilie der Nukleotidepyrophosphatasen und Phosphodiesterasen, insbesondere Autotaxin bewirken. Die erfindungsgemäßen Verbindungen zeigen bevorzugt eine vorteilhafte biologische Aktivität, die in den, zum Beispiel hierin beschrieben Test, leicht nachweisbar ist. In derartigen Tests zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Generell können alle soliden und nicht soliden Tumore mit den Verbindungen der Formel I behandelt werden, wie z.B. die Monozytenleukämie, Hirn-, Urogenital-, Lymphsystem-, Magen-, Kehlkopf- Ovarial- und Lungenkarzinom, darunter Lungenadenokarzinom und kleinzelliges Lungenkarzinom. Zu weiteren Beispielen zählen Prostata-, Bauchspeicheldrüsen- und Brustkarzinom.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch eine Inhibierung einer oder mehrerer Nukleotidepyrophosphatasen und/oder Phosphodiesterasen, insbesondere Autotaxin, beeinflusst werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine vorteilhafte Wirkung aufweisen.

Der Wirt oder Patient kann jeglicher Säugerspezies angehören, z. B. einer Primatenspezies, besonders Menschen; Nagetieren, einschließlich Mäusen, Ratten und Hamstern, Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Sensitivität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Zellmigration zu inhibieren oder die zelluläre Sekretion von angiogenesefördernden Substanzen zu blockieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten, wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden können.

### STAND DER TECHNIK

Verbindungen, die zur Hemmung von Autotaxin fähig sind, sind in Peng et al. Bioorganic & Medicinal Chemistry Letters (17, 2007, Seite 1634-1640) beschrieben. Die dort beschriebenen Verbindungen stellen Lipid-Analoga dar, welche strukturell keine Gemeinsamkeiten mit den erfindungsgemäßen Verbindungen aufweisen Niedermolekulare Autotaxin-Hemmer sind bekannt aus Parrill et al., Bioorganic & Medicinal Chemistry 16 (2008), Seiten 1784-1795. Autotaxin-Hemmer mit ähnlicher Struktur werden in WO2009/046841 beschrieben.

### ZUSAMMENFASSUNG DER ERFINDUNG

Die Erfindung betrifft Verbindungen der Formel I 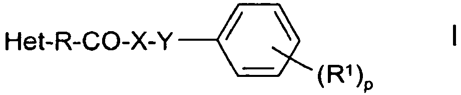 worin
- R¹: H, A, Hal, OR³, N(R³)₂, N=CR³N(R³)₂, SR³, NO₂, CN, COOR³, CON(R³)₂, NR³COA, NR³SO₂A, SO₂N(R³)₂, S(O)ₘA, -[C(R³)₂]ₙN(R³)₂, O[C(R³)₂)ₚN(R³)₂, S[C(R³)₂]ₙN(R³)₂, -NR³[C(R³)₂]ₙN(R³)₂, NHCON(R³)₂, CON(R³)_{2,} CONR³[C(R³)₂]ₙN(R³)₂ oder COA,
- R³: H oder A,
- X: O, NH oder CH₂,
- Y: CH₂, CH₂O oder fehlt,
R 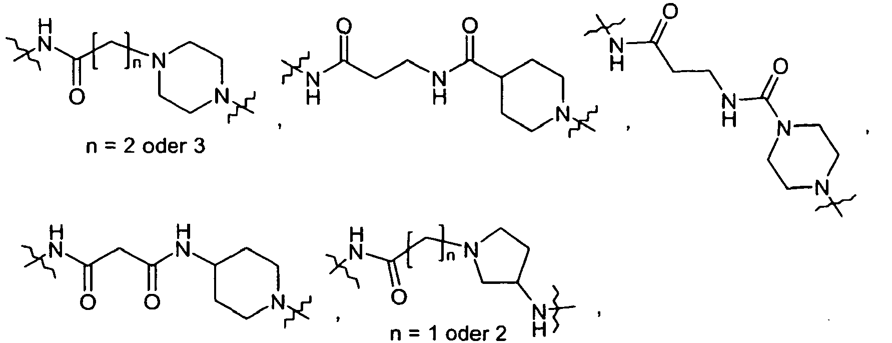 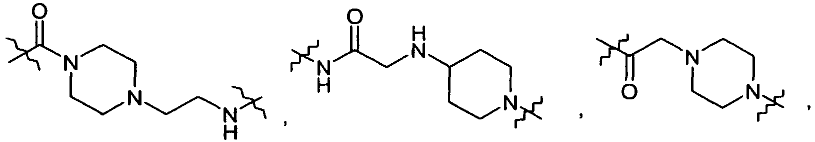 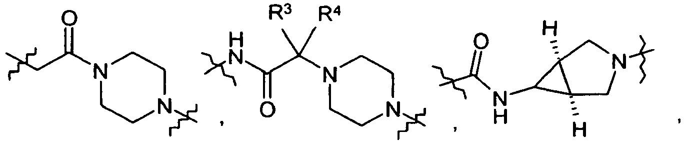 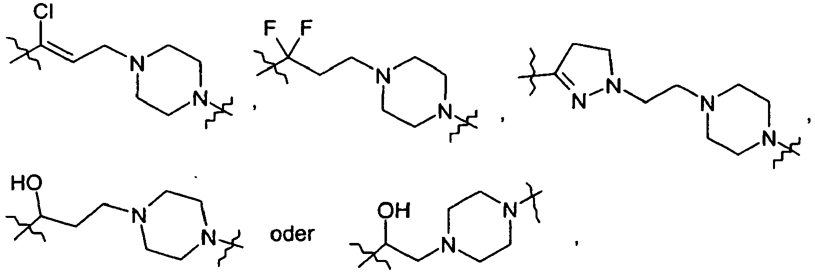
- R⁴: H, A oder Phenyl,
Het 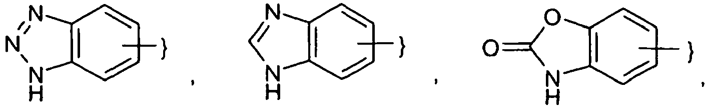 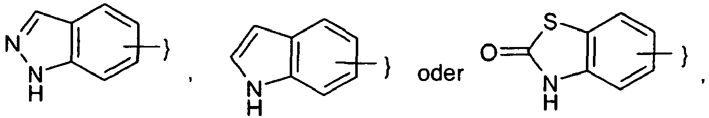
- A: unverzweigtes oder verzweigtes Alkyl mit 1-10 C-Atomen, worin 1-7 H-Atome durch OH, F, Cl und/oder Br ersetzt sein können, und/oder worin eine oder zwei CH₂-Gruppen durch O, NH und/oder S ersetzt sein können, oder cyclisches Alkyl mit 3-7 C-Atomen,
- Hal: F, Cl, Br oder I,
- n: 0, 1, 2 oder 3,
- m: 0, 1 oder 2,
- p: 0, 1, 2, 3, 4 oder 5,
bedeuten,
sowie ihre pharmazeutisch verwendbaren Salze und Stereoisomere, einschließlich deren Mischungen in allen Verhältnissen.

Verbindungen der Formel I bedeuten auch deren optisch aktiven Formen (Stereoisomeren), Tautomere, Polymorphe, Enantiomeren, Racemate, Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüber hinaus bedeutet der Ausdruck "therapeutisch wirksame Menge" eine Menge, die, verglichen zu einem entsprechenden Subjekt, das diese Menge nicht erhalten, hat folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfasst auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Patentansprüchen sowie ihrer pharmazeutisch verwendbaren Salze, und Stereoisomeren, dadurch gekennzeichnet, dass man
a) zur Herstellung von Verbindungen der Formel I, worin
   R 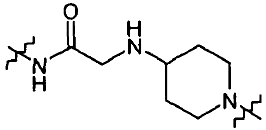 bedeutet,
   eine Verbindung der Formel II

   Het-NH-CO-CH₂-L II

   worin
   Het die in Anspruch 1 angegebene Bedeutung hat, und L Cl oder Br bedeutet,
   mit einer Verbindung der Formel III 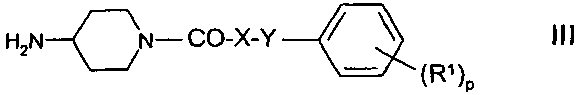 worin
   X, Y, R¹ und p die in Anspruch 1 angegebenen Bedeutungen haben,
   umsetzt,
   oder
b) zur Herstellung von Verbindungen der Formel I, worin
   R 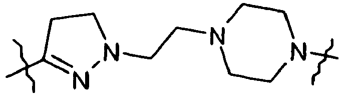 bedeutet,
   eine Verbindung der Formel IV 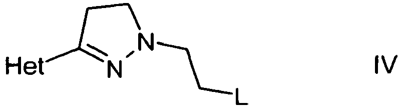 worin
   Het die in Anspruch 1 angegebene Bedeutung hat, und L Cl oder Br bedeutet,
   mit einer Verbindung der Formel V 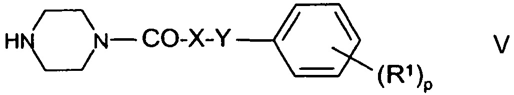 worin
   X, Y, R¹ und p die in Anspruch 1 angegebenen Bedeutungen haben,
   umsetzt,
   oder
c) zur Herstellung von Verbindungen der Formel I, worin
   R 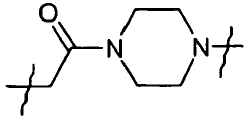 bedeutet,
   eine Verbindung der Formel VI

   Het-CH₂-CO-L VI

   worin
   Het die in Anspruch 1 angegebene Bedeutung hat, und L Cl, Br, I oder eine freie oder reaktionsfähig funktionell abgewandelte OH-Gruppe bedeutet,
   mit einer Verbindung der Formel V
   umsetzt,
   oder
d) zur Herstellung von Verbindungen der Formel I, worin
   R 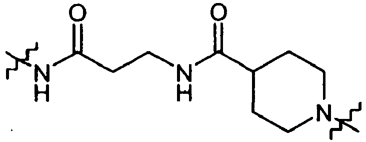 bedeutet,
   eine Verbindung der Formel VII 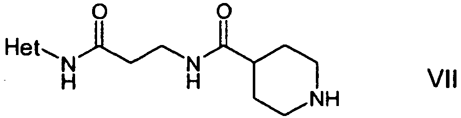 worin
   Het die in Anspruch 1 angegebene Bedeutung hat,
   mit einer Verbindung der Formel VIII 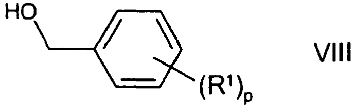 worin
   R¹ und p die in Anspruch 1 angegebenen Bedeutungen haben,
   und einer Verbindung ausgewählt aus der Gruppe Carbonyldiimidazol, Phosgen, Diphosgen, Triphosgen
   umsetzt,
   oder
e) zur Herstellung von Verbindungen der Formel I, worin
   R 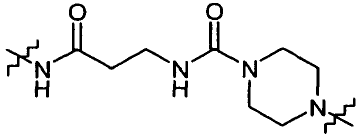 bedeutet,
   eine Verbindung der Formel IX 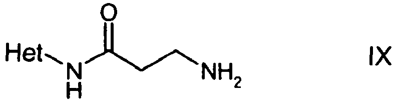 worin
   Het die in Anspruch 1 angegebene Bedeutung hat,
   mit einer Verbindung der Formel V
   und einer Verbindung ausgewählt aus der Gruppe Carbonyldiimidazol, Phosgen, Diphosgen, Triphosgen,
   umsetzt,
   oder
f) zur Herstellung von Verbindungen der Formel I, worin
   R 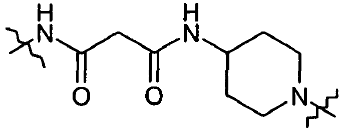 bedeutet,
   eine Verbindung der Formel X

   Het-NH₂ X

   worin
   Het die in Anspruch 1 angegebene Bedeutung hat,
   mit einer Verbindung der Formel XI 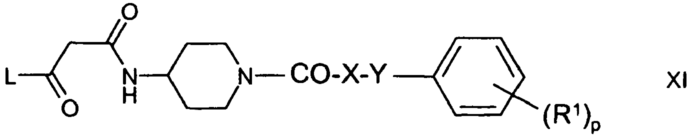 worin
   X, Y, R¹, p die in Anspruch 1 angegebenen Bedeutungen haben,
   und L Cl, Br, I oder eine freie oder reaktionsfähig funktionell abgewandelte OH-Gruppe bedeutet,
   umsetzt,
und/oder eine Base oder Säure der Formel I in eines ihrer Salze umwandelt.

A bedeutet Alkyl und ist bevorzugt unverzweigt (linear) oder verzweigt, und hat 1, 2, 3, 4, 5, 6, 7, 8, 9 oder 10 C-Atome. Alkyl bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

Alkyl bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. Alkyl bedeutet auch Cycloalkyl.

Cycloalkyl bedeutet vorzugsweise Cyclopropyl, Cyclobutyl, Cylopentyl, Cyclohexyl oder Cycloheptyl.

Hal bedeutet vorzugsweise F, Cl oder Br, aber auch I, besonders bevorzugt Br oder Cl.

R¹ bedeutet vorzugsweise Hal.

R³ bedeutet vorzugsweise H oder Methyl.

X bedeutet vorzugsweise O der CH₂.

Y bedeutet vorzugswesie CH₂ oder CH₂O.

p bedeutet vorzugsweise 1, 2 oder 3, ferner 4 oder 5.

n bedeutet vorzugsweise 0, 1, 2 oder 3.

Für die gesamte Erfindung gilt, dass sämtliche Reste, die mehrfach auftreten, wie z.B. R, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Einige bevorzugte Gruppen von Verbindungen können durch die folgenden Teilformeln la bis le ausgedrückt werden, die der Formel I entsprechen und worin die nicht näher bezeichneten Reste die bei der Formel I angegebene Bedeutung haben, worin jedoch

| | | |
|---|---|---|
| in Ia | R¹ | Hal bedeutet; |
| in Ib | X | O oder CH₂ bedeutet; |
| in Ic | Y | CH₂ oder CH₂O bedeutet; |
| in Id | P | 1, 2 oder 3 bedeutet; |
| in Ie | R¹ | Hal, |
| | X | O oder CH₂, |
| | Y | CH₂ oder CH₂O, |
| | R | |
| 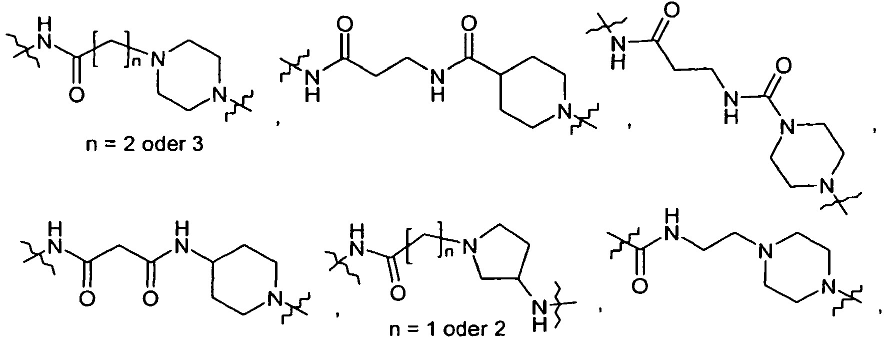 | | |
| 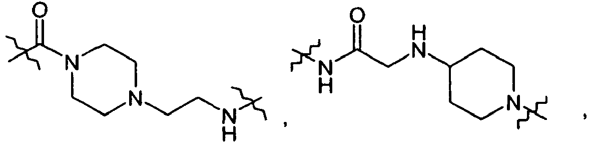 | | |
| 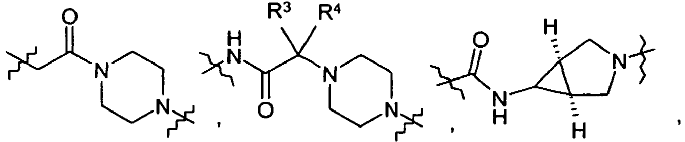 | | |
| 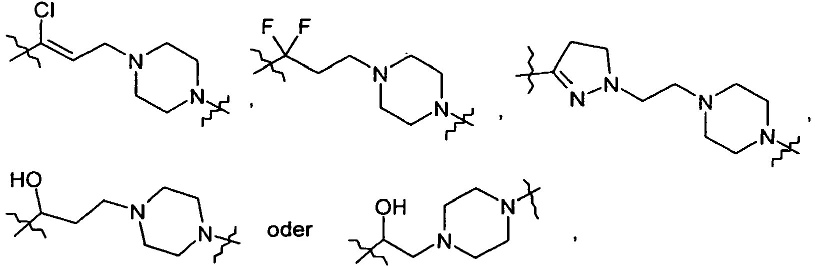 | | |
| | Het | |
| 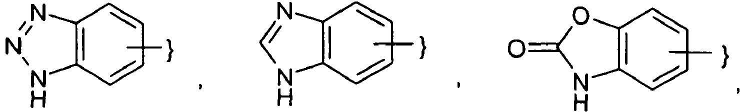 | | |
| 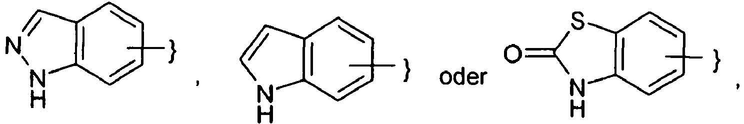 | | |
| A | unverzweigtes oder verzweigtes Alkyl mit 1-10 C-Atomen, worin 1-7 H-Atome F und/oder Cl ersetzt sein können, | |
| Hal | F, Cl, Br oder I, | |
| P | 1, 2 oder 3, | |
| | bedeuten, | |

sowie ihre pharmazeutisch verwendbaren Salze und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so dass man sie aus dem Reaktionsgemisch nicht isoliert, sondern sofort weiter zu den Verbindungen der Formel I umsetzt.

Die Ausgangsverbindungen der Formeln II, III, IV, V, VI, VII, VIII, IX, X und XI sind in der Regel bekannt. Sind sie neu, so können sie aber nach an sich bekannten Methoden hergestellt werden.

Die Edukte sind im Allgemeinen auch kommerziell erhältlich.

In den Verbindungen der Formel II, IV, VI, XI bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Verbindungen der Formel I können vorzugsweise erhalten, werden, indem man eine Verbindung der Formel II mit einer Verbindung der Formel III umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°, ganz besonders bevorzugt zwischen 15 und 35°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt ist Pyridin, Acetonitril, Dichlormethan und/oder DMF.

Verbindungen der Formel I können weiterhin vorzugsweise erhalten werden, indem man eine Verbindung der Formel IV mit einer Verbindung der Formel V umsetzt unter Bedingungen wie oben beschrieben.

Die Umsetzung erfolgt vorzugsweise in Acetonitril bei 100°C unter Zusatz von NaHCO₃.

Verbindungen der Formel I können weiterhin vorzugsweise erhalten werden, indem man eine Verbindung der Formel VI mit einer Verbindung der Formel V umsetzt. In den Verbindungen der Formel VI bedeutet L vorzugsweise OH. Zur Umsetzung wird die Carboxygruppe vorzugsweise in einen Aktivester überführt.

Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid.

Die Reaktion gelingt vorzugsweise in Gegenwart eines Dehydratisierungsmittels, wie z.B. eines Carbodiimids wie N,N'-Dicyclohexylcarbodiimid ("DCCI"), 1,1'-Carbonyl-diimidazol (CDI) oder N-3-Dimethylaminopropyl-N'-ethyl-carbodiimid ("DAPECI"), ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin.

Die Umsetzung erfolgt in der Regel in einem inerten Lösungsmittel.

Die Reaktionszeit liegt je nach den angewendeten Bedingungen zwischen einigen Minuten und 14 Tagen, die Reaktionstemperatur zwischen etwa -15° und 150°, normalerweise zwischen -5° und 90°, besonders bevorzugt zwischen 20° und 60°C.

Die Umsetzung erfolgt vorzugsweise in DMF bei Raumtemperatur und vorzugsweise unter Zusatz von N-Methylmorpholin.

Verbindungen der Formel I können weiterhin vorzugsweise erhalten werden, indem man eine Verbindung der Formel VII mit einer Verbindung der Formel VIII und einer Verbindung ausgewählt aus der Gruppe Carbonyldiimidazol, Phosgen, Diphosgen, Triphosgen,
umsetzt.

Die Umsetzung erfolgt in einem inerten Lösungsmittel und unter Bedingungen wie oben beschrieben. Vorzugsweise erfolgt die Reaktion in DMF bei Raumtemperatur und unter Zusatz einer Carbonylkomponente wie CDI.

Verbindungen der Formel I können weiterhin vorzugsweise erhalten werden, indem man eine Verbindung der Formel IX mit einer Verbindung der Formel V und einer Verbindung ausgewählt aus der Gruppe Carbonyldiimidazol, Phosgen, Diphosgen, Triphosgen,
umsetzt.

Die Umsetzung erfolgt in einem inerten Lösungsmittel und unter Bedingungen wie oben beschrieben. Vorzugsweise erfolgt die Reaktion in DMF bei Raumtemperatur und unter Zusatz einer Carbonylkomponente wie CDI und einer Base wie Triethylamin.

Verbindungen der Formel I können weiterhin vorzugsweise erhalten werden, indem man eine Verbindung der Formel VX mit einer Verbindung der Formel XI umsetzt.

Die Umsetzung erfolgt vorzugsweise unter Bedingungen wie die Umsetzung der Verbindung der Formel VI mit einer Verbindung der Formel V.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, dass man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, dass man die freie Basenform mit einer ausreichenden Menge der gewünschten Säure in Kontakt bringt, wodurch man auf übliche Weise das Salz darstellt. Die freie Base lässt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, dass man die freie Säureform mit einer ausreichenden Menge der gewünschten Base in Kontakt bringt, wodurch man das Salz auf übliche Weise darstellt. Die freie Säure lässt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfasst die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, dass unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste pharmazeutische Formulierungen können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepasste pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepassten pharmazeutischen Formulierungen gehören Augentropfen, wobei der Wirkstoff in einem geeigneten Träger, insbesondere einem wässrigen Lösungsmittel, gelöst oder suspendiert ist.

An die topische Applikation im Mund angepasste pharmazeutische Formulierungen umfassen Lutschtabletten, Pastillen und Mundspülmittel.

An die rektale Verabreichung angepasste pharmazeutische Formulierungen können in Form von Zäpfchen oder Einläufen dargereicht werden.

An die nasale Verabreichung angepasste pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste pharmazeutische Formulierungen können als Pessare, Tampons, Cremes, Gele, Pasten, Schäume oder Sprayformulierungen dargereicht werden.

Zu den an die parenterale Verabreichung angepassten pharmazeutischen Formulierungen gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, dass die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im Allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es lässt sich annehmen, dass ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen, und einer wirksamen Menge eines weiteren Arzneimittelwirkstoffs gelöst oder in lyophilisierter Form vorliegt.

Bevorzugt aber nicht ausschliesslich werden die Arzneimittel der Tabelle 1 mit den Verbindungen der Formel I kombiniert. Eine Kombination der Formel I und Arzneimitteln der Tabelle I kann auch mit Verbindungen der Formel VI kombiniert werden.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson |
| | Tetraplatin | Matthey) |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche Ethinylcytidin (Taiho) |
| | Idatrexate | |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron Irinotecan (CPT-11) | Gimatecan (Sigma- Tau) |
| | | Diflomotecan (Beaufour-Ipsen) |
| | 7-Ethyl-10-hydroxycamptothecin | |
| | | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharrna) | |
| | | |
| Antitumor-Antibiotik | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubici | |
| | Mitoxantron (Novantron) | |
| Antimitotische Mitte | Paclitaxel | SB 408075 |
| | Docetaxel | (GlaxoSmithKline) |
| | Colchicin | E7010 (Abbott) |
| | Vinblastin | PG-TXL (Cell Therapeutics) |
| | Vincristin | IDN 5109 (Bayer) |
| | Vinorelbin | A 105972 (Abbott) |
| | Vindesin | A 204197 (Abbott) |
| | Dolastatin 10 (NCI) | LU 223651 (BASF) |
| | Rhizoxin (Fujisawa) | D 24851 (ASTA Medica) |
| | Mivobulin (Warner-Lambert) | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | IDN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrug (OXIGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransferase Inhibitoren | Arglabin (NuOncology Labs) | Tipifarnib (Johnson & Johnson) |
| | Ionafarnib (Schering-Plough | |
| | BAY-43-9006 (Bayer) | Perillylalkohol (DOR BioPharma) |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransfe se-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidredul ase-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | Galliummaltolat (Titan) | Didox (Molecules for Health |
| | Triapin (Vion) | |
| | | |
| TNF-alpha-Agonisten/Antagon ten | Virulizin (Lorus Therapeutics | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | | |
| | p21-RAS-Impfstoff (GemVa: | |
| | | |
| Hormonelle und antihormonelle Mitt | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologie: | |
| | Motexafin-Gadolinium (Pharmacyclics) | Lutetium-Texaphyrin (Pharmacyclics) |
| | | Hypericin |
| | | |
| Tyrosinkinase-Inhibitoren | Imatinib (Novartis) | Kahalid F (PharmaMar) |
| | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| Verschiedene Mitte | SR-27897 (CCK-A-Inhibitor, Sanofi-Synthelabo) | BCX-1777 (PNP-Inhibitor, BioCryst) |
| | Tocladesin (cyclisches-AMP Agonist, Ribapharm) | Ranpirnase (Ribonuclease-Stimulans, Alfacell) |
| | Alvocidib (CDK-Inhibitor, Aventis) | Galarubicin (RNA-Synthese Inhibitor, Dong-A) |
| | CV-247 (COX-2-Inhibitor, Iv Medical) | Tirapazamin (Reduktionsmittel, SRI International) |
| | P54 (COX-2-Inhibitor, Phytopharm) | |
| | | N-Acetylcystein (Reduktionsmittel, Zambon) |
| | CapCell™ (CYP450-Stimulans, Bavarian Nordic) | |
| | | R-Flurbiprofen (NF-kappaB-Inhibitor, Encore) |
| | GCS-IOO (gal3-Antagonist, GlycoGenesys) | |
| | | 3CPA (NF-kappaB-Inhibitor Biotech) |
| | G17DT-Immunogen (Gastrir | |
| | Inhibitor, Aphton) | Seocalcitol (Vitamin-D-Rezeptor-Agonist, Leo) |
| | Efaproxiral (Oxygenator, Allos Therapeutics) | |
| | | 131-I-TM-601 (DNA-Antagonist, TransMolecular |
| | PI-88 (Heparanase-Inhibitor Progen) | |
| | | Eflornithin (ODC-Inhibitor, ILEX Oncology) |
| | Tesmilifen (Histamin-Antagonist, YM BioSciences | |
| | | Minodronsäure (Osteoclasten-Inhibitor, Yamanouchi) |
| | Histamin (Histamin-H2-Rezeptor- Agonist, Maxim) | |
| | | |
| | Tiazofurin (IMPDH-Inhibitor, Ribapharm) | Indisulam (p53-Stimulans, Eisai) |
| | Cilengitid (Integrin-Antagonist, Merck KGaA) | Aplidin (PPT-Inhibitor, PharmaMar) |
| | SR-31747 (IL-1-Antagonist, Sanofi-Synthelabo) | Rituximab (CD20-Antikörpe Genentech) |
| | CCI-779 (mTOR-Kinase-Inhibitor, Wyeth) | Gemtuzumab (CD33-Antikörper, Wyeth Ayerst) |
| | Exisulind (PDE-V-Inhibitor, Cell Pathways) | PG2 (Hämatopoese-Verstärker, Pharmagenesis) |
| | CP-461 (PDE-V-Inhibitor, C Pathways) | Immunol™ (Triclosan-Oralspülung, Endo) |
| | AG-2037 (GART-Inhibitor, Pfizer) | Triacetyluridin (Uridin-Prodrug, Wellstat) |
| | WX-UK1 (Plasminogenaktivator-Inhibitor, Wilex) | SN-4071 (Sarkom-Mittel, Signature BioScience) |
| | | TransMID-107™ (Immunotoxin, KS Biomedix |
| | PBI-1402 (PMN-Stimulans, ProMetic LifeSciences) | |
| | | PCK-3145 (Apoptose-Förderer, Procyon) |
| | Bortezomib (Proteasom-Inhibitor, Millennium) | |
| | | Doranidazol (Apoptose-Förderer, Pola) |
| | SRL-172 (T-Zell-Stimulans, SR Pharma) | |
| | | CHS-828 (cytotoxisches Mittel, Leo) |
| | TLK-286 (Glutathion-S-Transferase-Inhibitor, Telik) | |
| | | trans-Retinsäure (Differentiator, NIH) |
| | PT-100 (Wachstumsfaktor-Agonist, Point Therapeutics | |
| | | MX6 (Apoptose-Förderer, MAXIA) |
| | Midostaurin (PKC-Inhibitor, Novartis) | |
| | | Apomin (Apoptose-Förderer ILEX Oncology) |
| | Bryostatin-1 (PKC-Stimulans GPC Biotech) | |
| | | Urocidin (Apoptose-Fördere Bioniche) |
| | CDA-II (Apoptose-Förderer, Everlife) | |
| | | Ro-31-7453 (Apoptose-Förderer, La Roche) |
| | SDX-101 (Apoptose-Förderer, Salmedix) | |
| | | Brostallicin (Apoptose-Förderer, Pharmacia) |
| | Ceflatonin (Apoptose-Förderer, ChemGenex) | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharma) |
| | Methotrexat | DMDC (Hoffmann-La Roche |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothecin | |
| | | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharrna) | |
| Antitumor-Antibiotik | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubici | |
| | Mitoxantron (Novantron) | |
| | | |
| Antimitotische Mitte | Paclitaxel | SB 408075 (GlaxoSmithKline) |
| | Docetaxel | |
| | Colchicin | E7010 (Abbott) |
| | Vinblastin | PG-TXL (Cell Therapeutics) |
| | Vincristin | IDN 5109 (Bayer) |
| | Vinorelbin | A 105972 (Abbott) |
| | Vindesin | A 204197 (Abbott) |
| | Dolastatin 10 (NCI) | LU 223651 (BASF) |
| | Rhizoxin (Fujisawa) | D 24851 (ASTA Medica) |
| | Mivobulin (Warner-Lambert) | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | IDN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase Inhibitoren | Pemetrexed (Eli Lilly) | Nolatrexed (Eximias) |
| | ZD-9331 (BTG) | CoFactor™ (BioKeys) |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransferase Inhibitoren | Arglabin (NuOncology Labs) | Tipifarnib (Johnson & Johnson) |
| | lonafarnib (Schering-Plough | |
| | BAY-43-9006 (Bayer) | Perillylalkohol (DOR BioPharma) |
| | | |
| Pumpen-Inhibitorer | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransfe se-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidreduk ase-Inhibitoren | Neovastat (Aetema Laboratories) | CMT -3 (CollaGenex) |
| | | BMS-275291 (Celltech) |
| | Marimastat (British Biotech) | Tezacitabin (Aventis) |
| | Galliummaltolat (Titan) | Didox (Molecules for Health |
| | Triapin (Vion) | |
| | | |
| TNF-alpha-Agonisten/Antagon ten | Virulizin (Lorus Therapeutics | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | | |
| | LGD-1550 (Liqand) | |
| Immunmodulatorer | Interferon | Dexosom-Therapie (Anosys |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | p21-RAS-Impfstoff (GemVa: | |
| | | |
| Hormonelle und antihormonelle Mitt | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologies | |
| | Motexafin-Gadolinium (Pharmacyclics) | Lutetium-Texaphyrin (Pharmacyclics) |
| | | Hypericin |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| | | |
| Verschiedene Mitte | SR-27897 (CCK-A-Inhibitor Sanofi-Synthelabo) | BCX-1777 (PNP-Inhibitor, BioCryst) |
| | Tocladesin (cyclisches-AMP Agonist, Ribapharm) | Ranpirnase (Ribonuclease-Stimulans, Alfacell) |
| | Alvocidib (CDK-Inhibitor, Aventis) | Galarubicin (RNA-Synthese-Inhibitor, Dong-A) |
| | CV-247 (COX-2-Inhibitor, Iv Medical) | Tirapazamin (Reduktionsmittel, SRI International) |
| | P54 (COX-2-Inhibitor, Phytopharm) | |
| | | N-Acetylcystein (Reduktionsmittel, Zambon) |
| | CapCell™ (CYP450-Stimulans, Bavarian Nordic | |
| | | R-Flurbiprofen (NF-kappaB-Inhibitor, Encore) |
| | GCS-IOO (gal3-Antagonist, GlycoGenesys) | |
| | | 3CPA (NF-kappaB-Inhibitor, Active Biotech) |
| | G17DT-Immunogen (Gastrin-Inhibitor, Aphton) | |
| | | Seocalcitol (Vitamin-D-Rezeptor-Agonist, Leo) |
| | Efaproxiral (Oxygenator, Allos Therapeutics) | |
| | | 131-I-TM-601 (DNA-Antagonist, TransMolecular) |
| | PI-88 (Heparanase-Inhibito Progen) | |
| | | Eflornithin (ODC-Inhibitor, ILEX Oncology) |
| | Tesmilifen (Histamin-Antagonist, YM BioScience | |
| | | Minodronsäure (Osteoclaste Inhibitor, Yamanouchi) |
| | Histamin (Histamin-H2-Rezeptor- Agonist, Maxim) | |
| | | Indisulam (p53-Stimulans, Eisai) |
| | Tiazofurin (IMPDH-Inhibitor Ribapharm) | |
| | | Aplidin (PPT-Inhibitor, PharmaMar) |
| | Cilengitid (Integrin-Antagonist, Merck KGaA) | |
| | | Rituximab (CD20-Antikörper Genentech) |
| | SR-31747 (IL-1-Antagonist, Sanofi-Synthelabo) | |
| | | Gemtuzumab |
| | CCI-779 (mTOR-Kinase-Inhibitor, Wyeth) | (CD33-Antikörper, Wyeth Ayerst) |
| | | PG2 (Hämatopoese-Verstärker, Pharmagenesis) |
| | Exisulind (PDE-V-Inhibitor, Cell Pathways) | |
| | | Immunol™ (Triclosan-Oralspülung, Endo) |
| | CP-461 (PDE-V-Inhibitor, Cell Pathways) | |
| | | Triacetyluridin (Uridin-Prodrug, Wellstat) |
| | AG-2037 (GART-Inhibitor, Pfizer) | |
| | | SN-4071 (Sarkom-Mittel, Signature BioScience) |
| | WX-UK1 (Plasminogenaktivator-Inhibitor, Wilex) | |
| | | TransMID-107™ (Immunotoxin, KS Biomedix) |
| | PBI-1402 (PMN-Stimulans, ProMetic LifeSciences) | PCK-3145 (Apoptose-Förderer, Procyon) |
| | Bortezomib (Proteasom-Inhibitor, Millennium) | Doranidazol (Apoptose-Förderer, Pola) |
| | SRL-172 (T-Zell-Stimulans, SR Pharma) | CHS-828 (cytotoxisches Mittel, Leo) |
| | TLK-286 (Glutathion-S-Transferase-Inhibitor, Telik) | trans-Retinsäure (Differentiator, NIH) |
| | PT-100 (Wachstumsfaktor-Agonist, Point Therapeutics | MX6 (Apoptose-Förderer, MAXIA) |
| | Midostaurin (PKC-Inhibitor, Novartis) | Apomin (Apoptose-Förderer, ILEX Oncology) |
| | Bryostatin-1 (PKC-Stimulans, GPC Biotech) | Urocidin (Apoptose-Förderer Bioniche) |
| | CDA-II (Apoptose-Förderer Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer, ChemGenex) | |

Bevorzugt werden die Verbindungen der Formel I mit den mit bekannten Antikrebsmitteln kombiniert:
Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).

"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethyl-propanoat, 4,4'-Dihydroxybenzophenon-2,4-dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxy-phenyl)retinamid und N-4-Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumomekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und *4-Desmethoxy-3-desamino-3-aziridinyl-4-methylsulfonyl-*daunorubicin (siehe WO 00/50032), was jedoch keine Einschränkung darstellen soll.

Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797. Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxy-phenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropyl-amino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxan-then-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzo-furyl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Amino-pyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Insbesondere bevorzugt ist die Verwendung der erfindungsgemäßen Verbindung zur Behandlung und Prophylaxe von Tumorerkrankungen.

Der Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der Tumor ist weiterhin vorzugsweise ausgewählt aus der Gruppe Lungenadenokarzinom, kleinzellige Lungenkarzinome, Bauchspeicheldrüsenkrebs, Ovarialkarzinom, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Unter einem anderen Aspekt umfasst die Erfindung ein zur Behandlung eines Patienten, der ein Neoplasma, wie einen Krebs, hat, durch Verabreichung einer Verbindung der Formel (I) in Kombination mit einem antiproliferativen Mittel. Geeignete antiproliferative Mittel umfassen die in Tabelle 1 bereitgestellten.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetet oder Dichlormethan, trennt ab, trocknet die organische Phase über Natriumsulfat, dampft ein und reinigt duch Chromatographie an Kieselgel und/oder durch Kristallisation. Rt-Werte werden per HPLC mit erwähnten Laufmitteln bestimmt.

| | |
|---|---|
| Massenspektrometrie (MS): | EI (Electronenstoß-Ionisation) M⁺ |
| | FAB (Fast Atom Bombardment) (M+H)⁺ |
| | ESI (Electrospray lonization) (M+H)⁺ |

APCI-MS (atmospheric pressure chemical ionization - mass spectrometry) (M+H)⁺

### (A) HPLC-Methode (unpolar)

Solvent A: Wasser + 0,1 % TFA
Solvent B: Acetonitril+ 0,08% TFA
Flow: 1,5 ml/min

| | |
|---|---|
| Gradient: | 0,0 min 20% B |
| | 5,0 min 100% B |
| | 5,5 min 100% B |
| | 6,0 min 20% B |
| | 6,5 min 20% B |

Säule: Chromolith Performance RP18e 100-3

### (B) HPLC/MS-Methode (polar)

Solvent A: Wasser + 0,05 % Ameisensäure
Solvent B: Acetonitril + 0,04 % Ameisensäure
Flow: 2,4 ml/min, Wellenlänge : 220nm

| | |
|---|---|
| Gradient: | 0,0 min 4 % B |
| | 2,8 min 100 % B |
| | 3,3 min 100% B |
| | 3,4 min 4% B |

Säule: Chromolith® Speed ROD RP-18e 50-4.6 mm

### (C) HPLC-Methode

Säule: Chromolith SpeedROD, 50 x 4.6 mm² (Best.Nr. 1.51450.0001) von Merck
Gradient: 5.0 min, t = 0 min, A:B = 95:5, t = 4.4 min: A:B = 25:75, t = 4.5 min bis t = 5.0 min: A:B = 0:100
Fluß: 3.00 ml/min
Laufmittel A: Wasser + 0,01% HCOOH (Ameisensäure)
Laufmittel B: Acetonitril + 0,01% HCOOH
Wellenlänge: 220 nm

### Beispiel 1

### Herstellung von 4-{2-[(1H-Benzotriazol-5-carbonyl)-amino]-ethyl}-piperazin-1-carbonsäure-3,5-dichlor-benzyl ester (5)

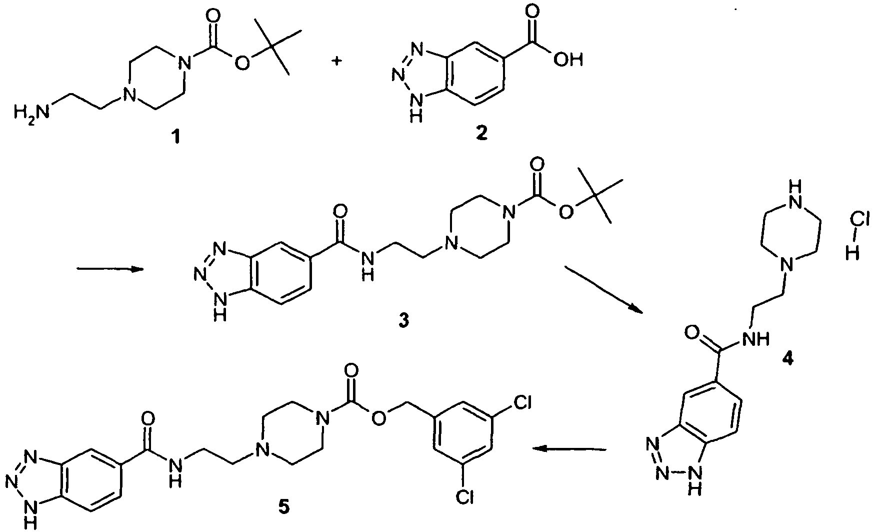

1.1 0.14 g (0.61 mmol) **1**, 0.12 g (0.74 mmol) **2** und 0.2 ml 4-Methylmorpholin werden in 6 ml DMF gelöst. Dann gibt man 0.14g (0.73 mmol) N-(3-Dimethylaminopropyl)-N'-ethylcarbodiimid x HCl (DAPECI) und 0.1 g (0.74 mmol) 1-Hydroxybenzotriazol (HOBt) hinzu. Es wird 18 h bei RT gerührt. Das Lösungsmittel wird am Rotationsverdampfer entfernt, mit Wasser verdünnt (100 mL) und 2x mit EE extrahiert. Die organische Phase wird über Magnesiumsulfat getrocknet, abfiltriert und zur Trockne eingedampft. Man erhält 0,21 g (92.3%) **3** als braune Kristalle.
1.2 0,21 g (0.56 mmol) **3** werden in 20 ml HCI/Isopropanol 5N gelöst und 2h bei RT gerührt. Zum vollständigen Ausfallen des Produktes wird der Ansatz mit 20 ml Ether versetzt. Das Produkt wird abgesaugt und bei 45° C im Vakuumtrockenschrank getrocknet. Man erhält 157 mg (80 %) braune Kristalle.
1.3 50 mg (0.28 mmol) 3,5-Dichlorbenzylalkohol und 60 mg (0.37 mmol) 1,1'- Carbonyldiimidazol (CDI) werden in 2 ml DMF gelöst und 3 h bei RT gerührt. Dann werden 0.1 g (0.32 mmol) **4** zugegeben und 18 h bei RT gerührt. Die Mischung wird mit Wasser gewaschen. Dann wird die organische Phase über Natriumsulfat getrocknet, abfiltriert und das Lösungsmittel im Vakuum eingedampft. Der Rückstand wird mittels präparativer HPLC aufgereinigt. Man erhält 62 mg (46%) 5 als hellbraune Kristalle.

### Beispiel 2

### Herstellung von 4-{[(2-Oxo-2,3-dihydro-benzoxazol-6-ylcarbamoyl)-methyl]-amino}-piperidin-1-carbonsäure-3,5-dichlor-benzylester (11)

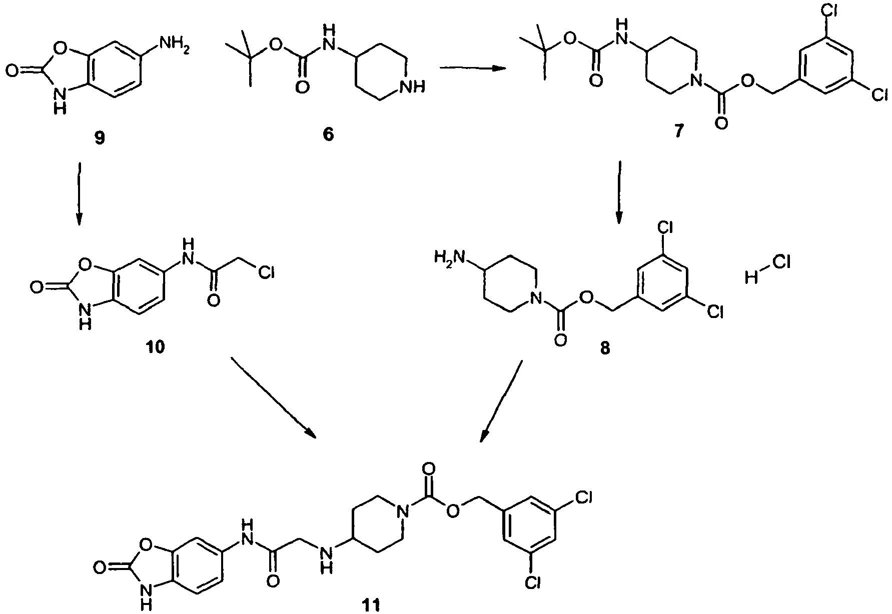

2.1 169,0 g (1.13 mol) **9** und 470 ml Triethylamin (3.39 mol) werden in 2 l Methylenchlorid (CH₂Cl₂) vorgelegt. Unter Eiskühlung gibt man so eine Lösung von 128 g (1.13 mol) Chloracetylchlorid in 1l CH₂Cl₂ hinzu, dass 8 °C Innentemperatur nicht überschritten werden. Dann wird 20 h unter Rückfluss gekocht. Nach Abkühlen des Reaktionsgemisches verrührt man es mit 3 I Wasser, wobei sich ein Niederschlag bildet. Der Niederschlag wird abgesaugt und mit Wasser und wenig Methanol gewaschen. Man erhält 183 g (71.7%) **10** als amorphe Festsubstanz.
2.2 0.5 g (2.8 mmol) 3,5-Dichlorbenzylalkohol und 0.55 g (3.4 mmol) 1,1' Carbonyldiimidazol (CDI) werden in 10 ml CH₂Cl₂ gelöst und 3 h bei RT gerührt. Dann werden 0.56 g (2.8 mmol) **6** zugegeben und 18 h bei RT gerührt. Die Mischung wird mit Wasser gewaschen. Dann wird die organische Phase über Natriumsulfat getrocknet, abfiltriert und das Lösungsmittel im Vakuum eingedampft. Der Rückstand wird mittels präparativer HPLC aufgereinigt. Man erhält 1.0 g (88%) 7 als weisse Kristalle.
2.3 1.0 g (2.48 mmol) 7 werden in 100 ml HCl/Isopropanol 5N gelöst und 2h bei RT gerührt. Zum vollständigen Ausfallen des Produktes wird der Ansatz mit 200 ml Ether versetzt. Das Produkt wird abgesaugt und bei 45° C im Vakuumtrockenschrank getrocknet. Man erhält 0.81 g (96 %) weisse Kristalle **8.**
2.4 0.92 g (2.44 mmol) **8** und 0.54 ml (3.9 mmol) NEt₃ werden in 10 ml DMF vorgelegt, dann werden 0.56 g (2.44 mmol) **10** hinzugegeben. Man rührt 48 h bei RT. Das Reaktionsgemisch wurde mit 30 ml Wasser versetzt, die ausgefallenen Kristalle abgesaugt. Die säulenchromatographische Trennung mit CH₂Cl₂/MeOH liefert 0.26 g (21%) **11** als braune Kristalle;
   RT [min] 3.68 (Methode A).

### Beispiel 3

### Herstellung von 4-[3-Hydroxy-3-(2-oxo-2,3-dihydro-benzoxazol-6-yl)-propyl]-piperazin-1-carbonsäure-3,5-dichlor-benzylester (13)

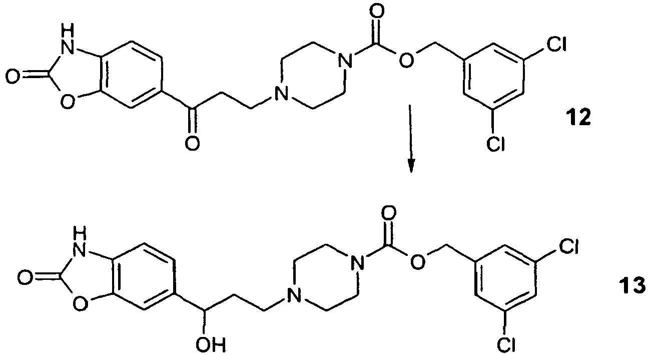

0,1 g **12** (0.21 mmol) werden in 5 ml Ethanol gelöst, dann gibt man 30.0 mg (0.79 mmol) Natriumborhydrid hinzu. Man rührt 14 h bei RT. Dann wird das Lösungsmittel im Vakuum eingedampft und der Rückstand mittels präparativer HPLC aufgereinigt. Man erhält 66 mg (65%) **13** als gelbliche Festsubstanz; RT [min] 3.57 (Methode A).

### Beispiel 4

### Herstellung von 4-[(Z)3-Chlor-3-(2-oxo-2,3-dihydro-benzoxazol-6-yl)-allyl]-piperazin-1-carbonsäure-4-chlor-benzylester (15)

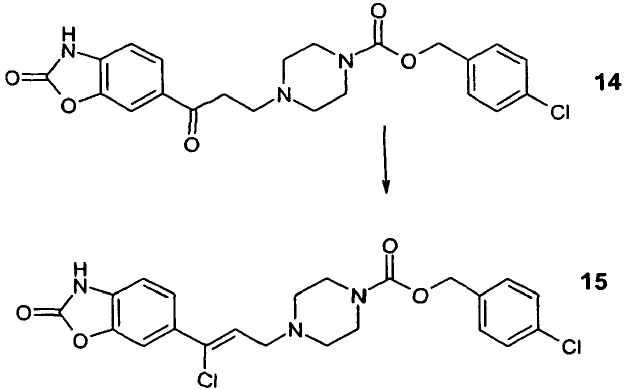

0,1 g **14** (0.23 mmol) werden mit 0.3 g (1.2 mmol) 1,2-Phenylendioxytrichlorphosphoran gemischt und 2 h auf 100° C erhitzt. Die dunkelbraune Schmelze wird mit 5 ml Methanol versetzt und 20 min im Ultraschallbad behandelt, wobei eine hellgelbe Festsubstanz ausfällt. Diese wird abgesaugt und nach Trocknen bei 45° C im Trockenschrank mittels präparativer HPLC aufgereinigt. Man erhält 5,8 mg (6%) **15** als gelbliche Festsubstanz; RT [min] 3.57 (Methode A).

### Beispiel 5

### Herstellung von 4-[3,3-Difluor-3-(2-oxo-2,3-dihydro-benzoxazol-6-yl)-propyl]-piperazin-1-carbonsäure-4-chlor-benzylester (17)

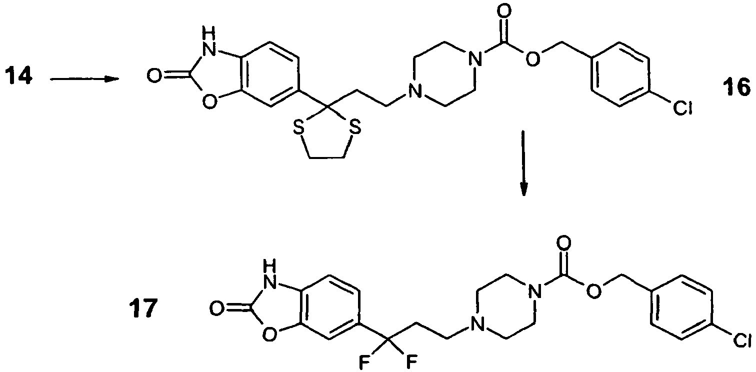

5.1 3,1 g (7,0 mmol) **14** werden in 2 ml Eisessig und 30 ml DCM gelöst. Unter Luftausschluss gibt man 1.3 ml (15 mmol) Ethandithiol hinzu, anschliessend tropft man unter Luftausschluss 0.98 ml (7 mmol) Bortrifluorid-Essigsäure-Komplex hinzu. Man rührt 72 h bei RT und versetzt dann mit 50 ml NaHCO₃-Lösung. Die ausgefallenen Kristalle werden abgesaugt. Es handelt sich um Edukt. Die organische Phase wird abgetrennt, mit MgSO₄ getrocknet. Die organische Phase wird abgetrennt, mit MgSO₄ getrocknet und im Vakuum eingedampft. Die säulenchromatographische Aufreinigung an Kieselgel mit Essigester ergiebt 0.1 g (3%) **16** als amorphe Festsubstanz.
5.2 57 mg (0.2 mmol) 1,3-Dibrom-5,5-dimethylhydantoin werden in 3 ml Dichlormethan vorgelegt. Unter Luftausschluss gibt man bei -78° C 0.23 ml (4 mmol) Pyridin-Fluorwasserstoff hinzu. Dann werden 100 mg (0.19 mmol) **16,** suspendiert in 5 ml Dichlormethan, hinzugegeben. Man rührt 20 min weiter. Dann wird die Kühlung entfernt und das Reaktionsgemisch mit 15 ml NaHCO₃-Lösung versetzt. Die organische Phase wird abgetrennt, mit MgSO4 getrocknet und im Vakuum eingedampft. Die Aufreinigung mittels präparativer HPLC ergibt 15 mg (17%) **17** als Festsubstanz; RT [min] 2.96 (Methode C).

### Beispiel 6

### Herstellung von 4-{2-[3-(2-Oxo-2,3-dihydro-benzoxazol-6-yl)-4,5-dihydro-pyrazol-1-yl]-ethyl}-piperazin-1-carbonsäure-3,5-dichlor-benzylester (21)

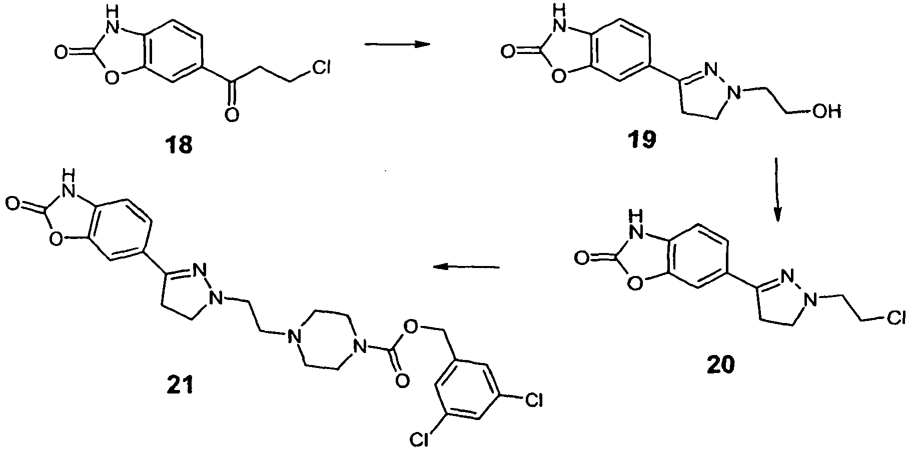

6.1 10,0 g (44 mmol) **18** werden in 100 ml Ethanol gelöst. Dann gibt man 4,0 g (52 mmol) Hydroxyethylhydrazin und anschliessend 7,0 ml (53 mmol) Triethylamin hinzu. Man rührt 2 h bei RT. Der entstandene gelbe Niederschlag wird abgesaugt und im Vakuumtrockenschrank bei 45° C getrocknet. Man erhält 4,1 g (37,4 %) **19** als gelbe Kristalle.
6.2 0,5 g (2,0 mmol) **19** werden in 10 ml DMF gelöst. Dann gibt man 0,5 ml (6,9 mmol) Thionychlorid hinzu und rührt 30 min bei RT. Das Reaktionsgemisch wird im Vakuum eingedampft. Der Rückstand wird mit 10 ml Acetonitril verrieben, die Kristalle abgesaugt und an der Luft getrocknet. Man erhält 0,32 g (60%) **20** als grünliche Kristalle.
6.3 0,12 g (0,4 mmol) **20**, 0,13 g (0,4 mmol) Piperazin-1-carbonsäure-3,5-dichlorbenzylester und 0,1 g (1,2 mmol) NaHCO₃ werden in 3 ml Acetonitril 16 h bei 100° C gerührt. Nach dem Abkühlen wird das Reaktionsgemisch mit 20 ml Wasser versetzt und zweimal mit CH₂Cl₂ extrahiert. Die organische Phase wird mit NaSO₄ getrocknet und im Vakuum eingedampft. Die säulenchromatographische Aufreinigung an Kieselgel mit Essigester/Methanol ergibt 21 mg (10%) **21** als gelbbraune Kristalle; RT [min] 2.80 (Methode A).

### Beispiel 7

### Herstellung von 4-(2-1H-Benzotriazol-5-yl-acetyl)-piperazin-1-carbonsäure-4-chlor-benzylester (24)

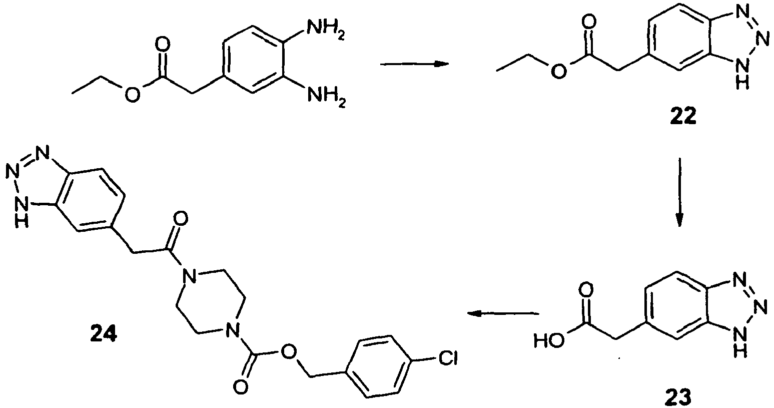

7.1 5,0 g (26 mmol) 3,4-Diaminophenylessigsäureethylester werden in 40 ml 50%iger Essigsäure gelöst und im Eisbad gekühlt. 2,7 g (39 mmol) Natriumnitrit in 20 ml Wasser werden so zugetropft, dass die Temperatur unter 10° C bleibt. Man rührt 3 h bei 10° - 20° C. Dann verdünnt man den Ansatz mit 200 ml Essigester und wäscht mit Wasser. Die organische Phase wird mit NaSO₄ getrocknet. und im Vakuum eingedampft. Man erhält 5,7 g (108%) **22** als braunes Öl (enthält noch etwas Lösemittel).
7.2 5,7 g (28 mmol) **22** werden in 25 ml Wasser und 10 ml EtOH gelöst und mit 75 ml 5%iger wässriger NaOH-Lösung versetzt. Man erhitzt 3 h unter Rückfluss. Nach Abkühlen wird im Vakuum eingedampft. Der Rückstand wird in 100 ml Wasser gelöst und mit 5-6 N HCl in Propanol auf pH 4 eingestellt. Die ausgefallenen Kristalle werden abgesaugt. Man erhält 3,3 g (56%) 23 als braune Kristalle.
7.3 220 mg (1,24 mmol) **23,** 362 mg (1,24 mmol) Piperazin-1-carbonsäure-4-chlorbenzylester-hydrochlorid, 168 mg (1,24 mmol) 1-Hydroxybenzotriazol (HOBt), 143 mg (1,24 mmol) N-Methylmorpholin und 238 mg (1,24 mmol) 3-Dimethylaminopropyl-carbodiimid-hydrochlorid (DAPECI) werden in 10 ml DMF 48 h bei RT gerührt.
   Dann wird das Reaktionsgemisch im Vakuum eingedampft. Die Aufreinigung mittels präparativer HPLC liefert 16 mg (3%) **24** als farblose Festsubstanz; RT [min] 3.73 (Methode A);
   ¹H-NMR (DMSO-d₆) δ [ppm] 7.86 (d, J = 8.5, 1H), 7.75 (d, J = 1.4, 1H), 7.46-7.36 (m, 4H), 7.34 (dd, J = 8.5,1.4, 1H), 5.10 (s, 2H), 3.95 (s, 2H), 3.65-3.30 (m, 7H).

### Beispiel 8

### Herstellung von 6-(2-{4-[3-(4-Chlor-phenoxy)-propionyl]-piperazin-1-yl}-acetyl)-3H-benzoxazol-2-on (27)

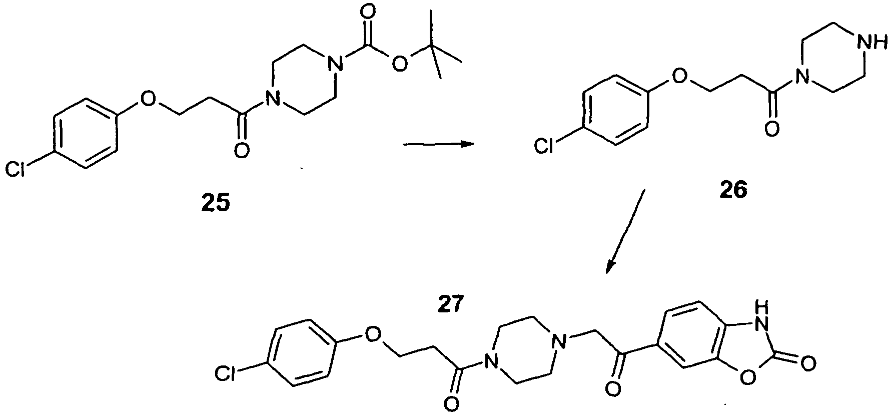

8.1 500 mg (2,5 mmol) 3-(4-Chlorphenoxy)-propionsäure, 464 mg (2,5 mmol) Boc-Piperazin und 253 mg (2,5 mmol) 4-Methylmorpholin werden in 3 ml DMF vorgelegt. Dann gibt man 478 mg (2,5 mmol) DAPECI und 337 mg (2,5 mmol) HOBt hinzu und rührt 16 h bei RT. Das Reaktionsgemisch wird auf Wasser gegossen und der entstandene Niederschlag abgesaugt und getrocknet. Man erhält 894 mg (97%) **25.**
8.2 894 mg (2,4 mmol) 25 werden in 10 ml 5-6N HCl in Propanol eingebracht und 0,5 h bei RT gerührt. Der dabei entstandene Niederschlag wird abgesaugt und getrocknet Man erhält 661 mg (89%) **26.**
8.3 104 mg (0,49 mmol) 6-(2-Chloracetyl)-3H-benzoxazolon (Synthese beschrieben unter Aktenzeichen 102007047737.8 beim Deutschen Patentamt) werden in 3 ml Acetonitril vorgelegt. Dann gibt man 150 mg (1,5 mmol) Triethylamin und 150 mg (0,49 mmol) **26** dazu und rührt 16 h bei RT und weitere 16 h bei 80° C. Der dabei entstandene Niederschlag wird abfiltriert und das Filtrat im Vakuum eingedampft. Die säulenchromatographische Aufreinigung an Kieselgel mit EE ergibt 6 mg (3%) **27;** RT [min] 3.04 (Methode A);
   ¹H-NMR (DMSO-d₆) δ [ppm] 11.66 (s, 1H), 7.90-7.84 (m, 2H),
   7.31 (d, J = 9.0, 2H), 7.19 (d, J = 7.6, 1 H), 6.95 (d, J = 9.0, 2H), 4.18 (t, J = 6.2, 2H), 3.86 (s, 2H), 3.62 - 3.30 (m, 8H), 2.80 (t, J = 6.2, 2H).

### Beispiel 9

### Herstellung von 4-[2-(1H-Benzotriazol-5-ylcarbamoyl)-ethylcarbamoyl]-piperidin-1-carbonsäure-4-chlor-benzylester (32)

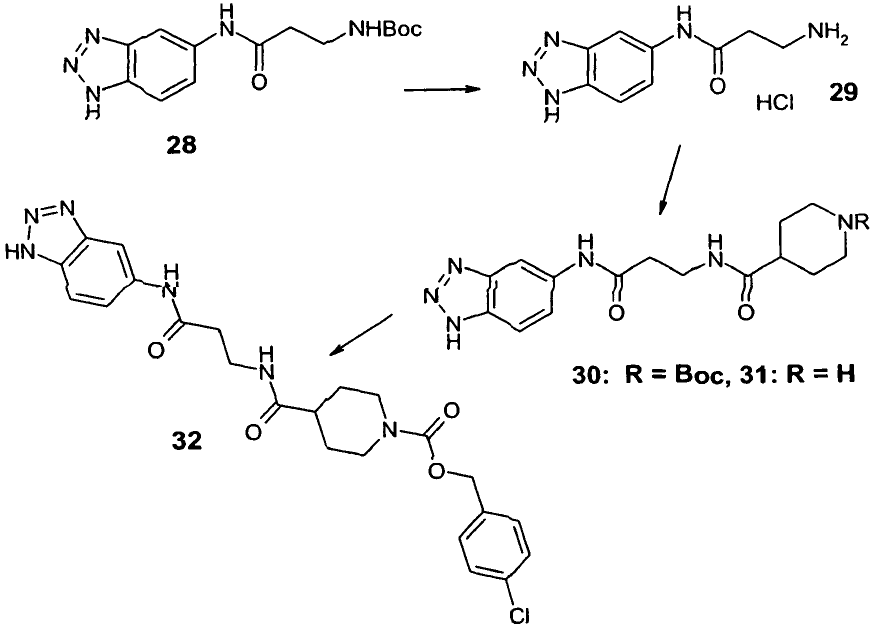

9.1 2,7 g (20 mmol) 5-Aminobenzotriazol, 3,8 g (20 mmol) 3-tert.-Butoxycarbonylaminopropionsäure, 3,5 g HOBt (26 mmol) und 4,2 g (22 mmol) DAPECI werden in 30 ml DMF gelöst und 16 h bei RT gerührt. Dann wird das Reaktionsgemisch im Vakuum eingedampft. Der Rückstand wird in 200 ml Essigester aufgenommen und 2 mal mit Wasser ausgeschüttelt. Die organische Phase wird mit MgSO₄ getrocknet und im Vakuum eingedampft. Die säulenchromatographische Aufreinigung an Kieselgel mit Essigester ergibt 5,7 g (93%) **28.**
9.2 5,7 g (19 mmol) **28** werden in 60 ml 6N HCl in Isopropanol gelöst und 1 h bei RT gerührt. Dann wird das Reaktionsgemisch im Vakuum eingedampft. Der Rückstand wird mit Methylenchlorid verrieben und abgesaugt. Man erhält 4,1 g (91%) **29** als hellbraune amorphe Festsubstanz.
9.3 0,97 g (4,0 mmol) **29,** 0,92 g (4,0 mmol) 1-Butoxycarbonylpiperidin-4-carbonsäure, 0,54 g HOBt (4,0 mmol) und 0,77 g (4,0 mmol) DAPECI und 0,55 ml Triethylamin (4,0 mmol) werden in 10 ml DMF gelöst und 16 h bei RT gerührt. Dann wird das Reaktionsgemisch im Vakuum eingedampft. Der Rückstand wird in 100 ml Essigester aufgenommen und zweimal mit Wasser ausgeschüttelt. Die organische Phase wird mit MgSO₄ getrocknet und im Vakuum eingedampft. Die säulenchromatographische Aufreinigung an Kieselgel mit Essigester ergibt 1,4 g (84%) **30.**
9.4 1,4 g (3,4 mmol) **30** werden in 20 ml 6 N HCl in Isopropanol gelöst und 1 h bei RT gerührt. Dann wird das Reaktionsgemisch im Vakuum eingedampft. Man erhält 1,1 g (93%) **31** als braune amorphe Festsubstanz.
9.5 81 mg (0,5 mmol) CDI und 78 mg (0,5 mmol) 4-Chlorbenzylalkohol werden in 3 ml DMF gelöst und 2 h bei RT gerührt. Dann gibt man 176 mg (0,5 mmol) **31** hinzu und rührt 16 h bei RT. Dann wird das Reaktionsgemisch im Vakuum eingedampft. Der Rückstand wird in 20 ml Essigester aufgenommen und zweimal mit Wasser ausgeschüttelt. Die organische Phase wird mit MgSO₄ getrocknet und im Vakuum eingedampft. Der Rückstand wird mit Ethanol kristallisiert. Man erhält 77 mg (32%) **32** als hellbraune Kristalle; RT [min] 3.36 (Methode C).

### Beispiel 10

### Herstellung von 4-[2-(1 H-Benzotriazol-5-ylcarbamoyl)-ethylcarbamoyl]-piperazin-1-carbonsäure-4-chlor-benzylester (33)

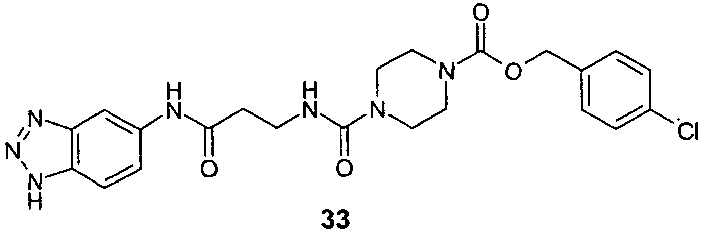

121 mg (0,5 mmol) **29** werden mit 0,035 ml Triethylamin in 3 ml DMF vorgelegt und mit 81 mg (0,5 mmol) CDI versetzt. Man rührt 1 h bei RT und gibt dann 146 mg (0,5 mmol) Piperazin-1-carbonsäure-4-chlorbenzylester-hydrochlorid und weitere 0,035 ml Triethylamin hinzu. Man rührt dann 2 h bei RT. Dann wird das Reaktionsgemisch im Vakuum eingedampft. Der Rückstand wird in 20 ml Essigester aufgenommen und zweimal mit Wasser ausgeschüttelt. Die organische Phase wird mit MgSO₄ getrocknet und im Vakuum eingedampft. Der Rückstand wird aus Ethanol umkristallisiert. Man erhält 81 mg (33%) **33** als hellbraune Kristalle; RT [min] 3.25 (Methode C).

### Beispiel 11

### Herstellung von 4-[2-(1H-Benzotriazol-5-ylcarbamoyl)-acetylaminol-piperidin-1-carbonsäure-4-chlor-benzyiester (39)

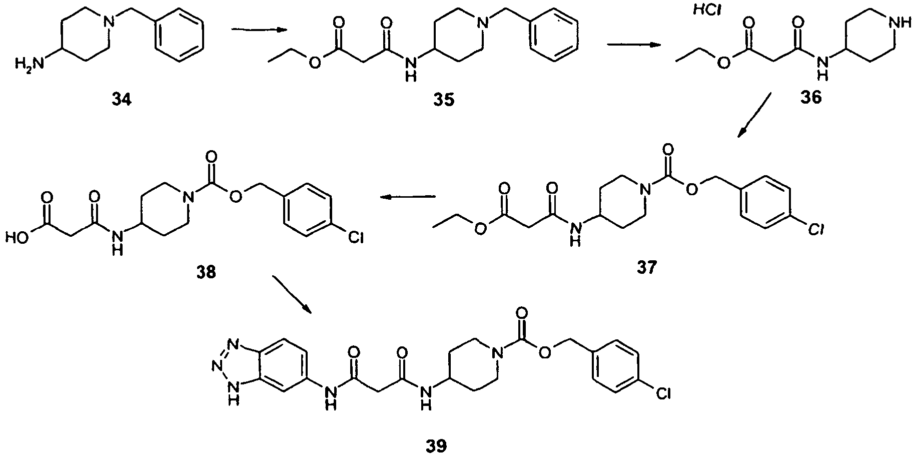

11.1 5,23 g (27,5 mmol) 34 und 3,8 ml (27,5 mmol) Triethylamin werden in 50 ml Methylenchlorid vorgelegt. Unter Eiskühlung tropft man 4,14 g (27,5 mmol) Malonsäureethylesterchlorid und rührt dann noch 1 h bei RT. Das Reaktionsgemisch wird mit Wasser extrahiert, die organische Phase dann abgetrennt, mit NaSO₄ getrocknet und im Vakuum eingedampft. Die säulenchromatographische Aufreinigung an Kieselgel mit Essigester ergibt 2,85 (34%) **35** als farblose Kristalle.
11.2 2,0 g (6,6 mmol) **35** werden in 20 ml THF und 20 ml Eisessig gelöst, mit 2g Pd/C-5% versetzt und hydriert. Es wird vom Katalysator abfiltriert. Man gibt 6N HCl hinzu und verdampft dann das Lösungsmittel im Vakuum. Man erhält 1,6g (97%) **36** als Festsubstanz.
11.3 1,5 g (10,5 mmol) 4-Chlorbenzylalkohol, 1,7 g (10,5 mmol) CDI werden in 10 ml DMF 2 h bei RT gerührt. Dann gibt man 1.04 ml (7,5 mmol) Triethylamin und 1,6 g (7,5 mmol) **36** hinzu und rührt 16 h bei RT. Dann gibt man das Reaktionsgemisch auf Wasser. Der entstandene Niederschlag wird abgesaugt und säulenchromatographisch an Kieselgel mit Petrolether/Essigester (1:1) aufgereinigt. Man erhält 1,46 g (51%) 37 als hellgelbe Kristalle.
11.4 1,46 g (3,8 mmol) **37** werden in 10 ml Ethanol gelöst, mit 3,0 ml wässriger 2N NaOH versetzt und 16 h bei RT gerührt. Man säuert die Lösung mit 1 N HCl an und rotiert ein. Der Rückstand wird mit Essigester aufgenommen und mit Wasser gewaschen. Die organische Phase wird mit NaSO4 getrocknet und einrotiert. Man erhält 0,47 g (35%) **38** als amorphe Festsubstanz.
11.5 0,47 g (1,32 mmol) **38,** 0,178 g (1,32 mmol) 5-Aminobenzotriazol, 0,28 g (1,46 mmol) DAPECI und 0,20 g (1,46 mmol) HOBt werden in 5 ml DMF gelöst und 16 h bei RT gerührt. Dann wird das Reaktionsgemisch im Vakuum eingedampft. Der Rückstand wird in 20 ml Essigester aufgenommen und 2 mal mit Wasser ausgeschüttelt. Die organische Phase wird mit MgSO₄ getrocknet. und im Vakuum eingedampft. Dabei fällt ein weisser Niederschlag aus, der abgesaugt und getrocknet wird. Man erhält 0,14 g (22%) **39** als farblose Festsubstanz; RT [min] 3.33 (Methode C).

Analog den vorstehenden Beispielen werden folgende Verbindungen erhalten
die nachstehenden Verbindungen

| Nr. | Struktur und/oder Name | RT [min] (Methode) |
|---|---|---|
| "A1" | 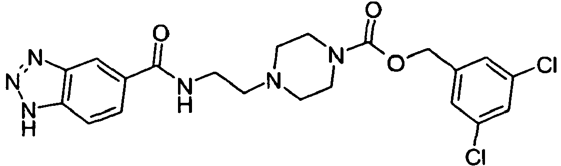 | 3.23 (A) |
| | 4-{2-[(1H-Benzotriazol-5-carbonyl)-amino]-ethyl}-piperazin-1-carbonsäure-3,5-dichlor-benzylester | |
| "A2" | 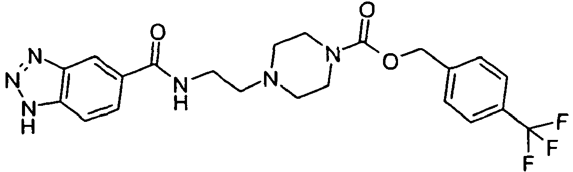 | 3.28 (A) |
| | 4-{2-[(1H-Benzotriazol-5-carbonyl)-amino]-ethyl}-piperazin-1-carbonsäure-4-trifluormethyl-benzyleste | |
| "A3" | 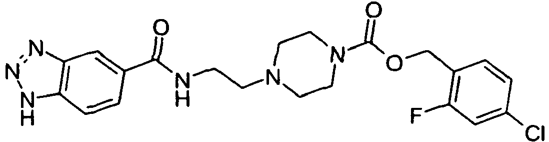 | 3.12 (A) |
| | 4-{2-[(1H-Benzotriazol-5-carbonyl)-amino]-ethyl}-piperazin-1-carbonsäure-4-chlor-2-fluor-benzylester | |
| ¹H-NMR (DMSO-d₆) δ [ppm] 8.61 (t, J = 5.6, 1H), 8.44 (s, 7.94 (s, 2H) 7.58-7.41 (m, 3H), 7.34-7.27 (m, 1H), 5.10 (s, 2H), 3.47-3.39 (m, 6H), 2.53 (t, J = 7.0, 2H), 2.47-2.38 (m, 4H) | | |
| "A4" | 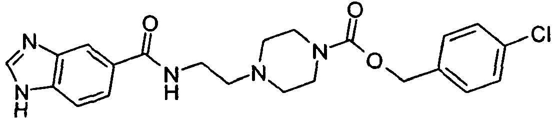 | 2.72 (A) |
| | 4-{2-[(1H-Benzimidazol-5-carbonyl)-amino]-ethyl}-piperazin-1-carbonsäure-4-chlor-benzylester Hydrochlorid | |
| ¹H-NMR (DMSO-d₆) δ [ppm] 9.75 (s, 1H), 8.37 (d, J = 1.0, 1H), 8.12 (dd, J 8.7, 1.0, 1H), 7.99 (d, J = 8.7, 1H), 7.44 (s, 4H), 5.14 (s, 2H), 4.25-4.10 (n, 2H), 3.73 (t, J = 7.0 Hz, 2H), 3.71 - 3.56 (m, 2H), 3.40-3.1 (m, 6H) | | |
| "A5" | 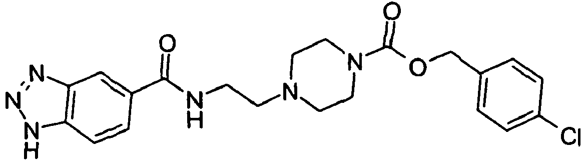 | 3.15 (A) |
| | 4-{2-[(1H-Benzotriazol-5-carbonyl)-amino]-ethyl}-piperazin-1-carbonsäure-4-chlor-benzylester | |
| "A6" | 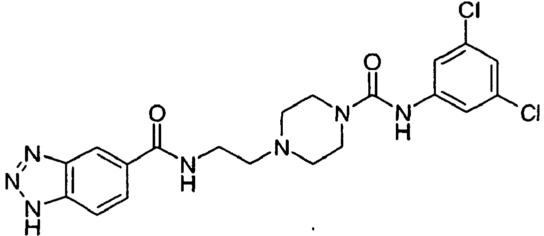 | 3.12 (A) |
| | 1H-Benzotriazol-5-carbonsäure-{2-[4-(3,5-dichlor-phenylcarbamoyl)-piperazin-1-yl]-ethyl}-amid | |
| "A7" | 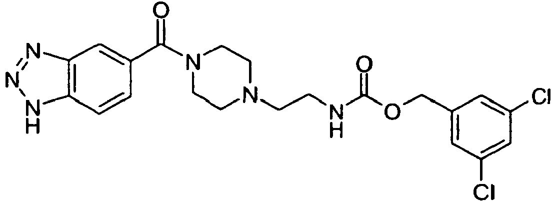 | 3.15 (A) |
| | {2-[4-(1H-Benzotriazol-5-carbonyl)-piperazin-1-yl]-ethyl}-carbaminsäure-3,5-dichlor-benzylester | |
| ¹H-NMR (DMSO-d₆) δ [ppm] 8.12 (s, 1H), 7.97 (d, J = 8.6 Hz, 1H), 7.56 (d, J = 8.6, 1H), 7.46-7.39 (m, 3H), 5.11 (s, 2H), 3.72-3.20 (m, 12H [identified in there: 3.52 (t, J = 5.8, 2H), 3.32 (t, J = 5.8, 2H)] | | |
| "A8" | 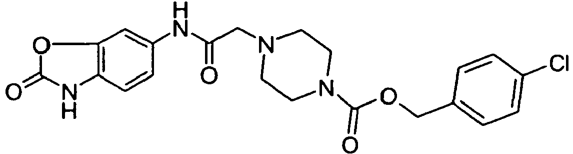 | 3.25 (B) |
| | 4-[(2-Oxo-2,3-dihydro-benzoxazol-6-ylcarbamoyl)-methyl]-piperazin-1-carbonsäure-4-chlor-benzyleste | |
| ¹H-NMR (DMSO-d₆) δ [ppm] 7.67 (d, J = 1.8, 1H), 7.40 (s, 4H), 7.20 (dd, J = 8.4, 1.9, 2H), 7.06 (d, J = 8.4, 1H), 5.10 (s, 2H), 4.18 (s, 2H), 3.68-3.04 (m, 7H) | | |
| "A9" | 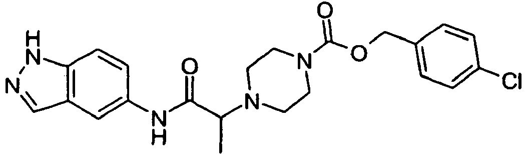 | 3.31 (B) |
| | 4-[1-(1H-Indazol-5-ylcarbamoyl)-ethyl]-piperazin-1-carbonsäure-4-chlor-benzylester | |
| "A10" | 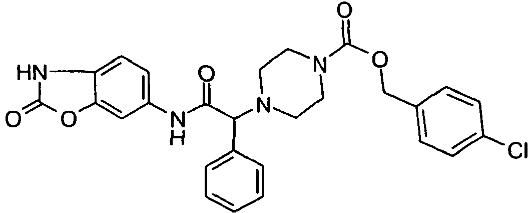 | 3.89 (B) |
| | 4-[(2-Oxo-2,3-dihydro-benzoxazol-6-ylcarbamoyl)-phenyl-methyl]-piperazin-1-carbonsäure-4-chlor-benzylester | |
| "A11" | 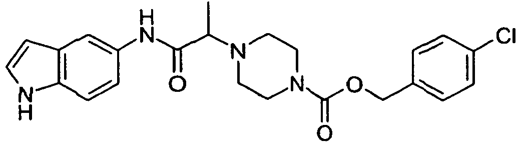 | 3.79 (B) |
| | 4-[1-(1H-Indol-5-ylcarbamoyl)-ethyl]-piperazin-1-carbonsäure-4-chlor-benzylester | |
| "A12" | 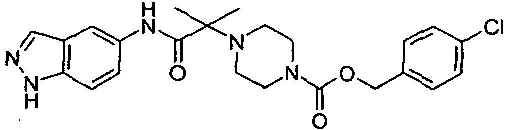 | 3.41 (A) |
| | 4-[1-(1H-Indazol-5-ylcarbamoyl)-1-methyl-ethyl]-piperazin-1-carbonsäure-4-chlor-benzylester | |
| "A13" | 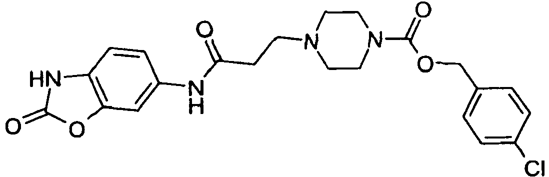 | 3.28 (B) |
| | 4-[2-(2-Oxo-2,3-dihydro-benzoxazol-6-ylcarbamoyl)-ethyl]-piperazin-1-carbonsäure-4-chlor-benzylester | |
| "A14" | 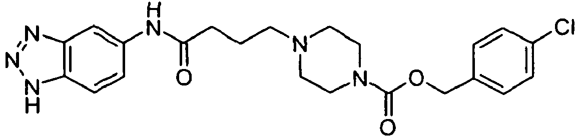 | 2.35 (C) |
| | 4-[3-(1H-Benzotriazol-5-ylcarbamoyl)-propyl]-piperazin-1-carbonsäure-4-chlor-benzylester | |
| "A15" | 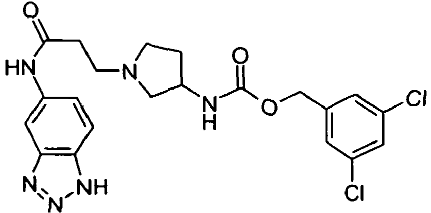 | 3.25 (A) |
| | {1-[2-(1H-Benzotriazol-5-ylcarbamoyl)-ethyl]-pyrrolidine 3-yl}-carbaminsäure-3,5-dichlor-benzylester | |
| ¹H-NMR (DMSO-d₆) δ [ppm] 10.31 (s, 1H), 8.36 (s, 1H), 7.86 (d, J = 8.9, 1H), 7.64 (s, 1H), 7.56 (s, 1H), 7.42 (s, 2H), 7.35 (d, J = 8.9, 1H), 5.02 (s, 2H), 4.05-3.89 (m, 2H), 2.86-2.68 (m, 4H), 2.68-2.56 (m, 2H), 2.47-2.31 (m, 2H), 2.19-2.02 (m, 1H), 1.66-1.56 (m, 1H) | | |
| "A16" | 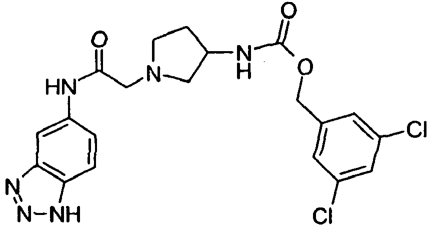 | 3.25 (A) |
| | {1-[(1H-Benzotriazol-5-ylcarbamoyl)-methyl]-pyrrolidir 3-yl}-crbaminsäure-3,5-dichlor-benzylester | |
| ¹H-NMR (DMSO-d₆) δ [ppm] 15.52 (s, 1H), 9.97 (s, 1H), 8.35 (s, 1H), 7.90 (s, 1H), 7.70 (d, J = 7.4, 1H), 7.61 - 7.35 (m, 4H), 5.04 (s, 2H), 4.05 (s, 1H 3.45-3.35 (m, 2H, covered by water), 2.89-2.77 (m, 2H), 2.63-2.53 (m, 2H) 2.22-2.12 (m, 1H), 1.72-1.63 (m, 1H) | | |
| "A17" | 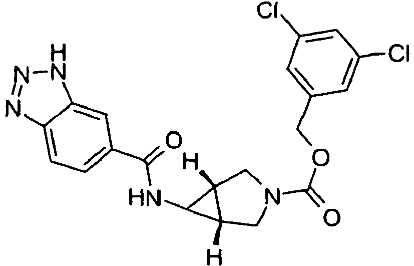 | 3.92 (A) |
| | (1R,5S)-6-[(3H-Benzotriazole-5-carbonyl)-amino]-3-aza-bicyclo[3.1.0]hexan-3-carbonsäure-3,5-dichlorbenzylester | |
| ¹H-NMR (DMSO-d₆) δ [ppm] 15.91 (s, 1H), 8.70 (d, J = 3.8, 1H), 8.42 (s, 1H), 7.91 (s, 2H), 7.56 (s, 1H), 7.43 (d, J = 1.6, 2H), 5.07 (d, J = 5.2, 2H), 3.67 (d, J = 10.7, 1H), 3.61 (d, J = 10.7, 1H), 3.54 (dd, J = 10.7, 2.6, 1H), 3.47 (dd, J = 10.7, 2.6, 1H), 2.63 (s, 1H), 1.97-1.82 (m, 2H) | | |
| "A18" | 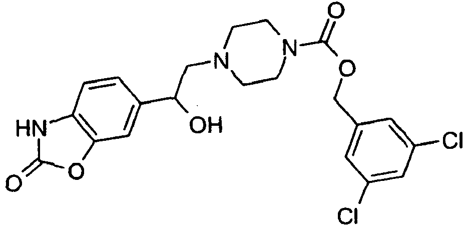 | 3.49 (A) |
| | 4-[2-Hydroxy-2-(2-oxo-2,3-dihydro-benzoxazol-6-yl)-ethyl]-piperazin-1-carbonsäure-3,5-dichlor-benzyleste | |
| ¹H-NMR (DMSO-d₆) δ [ppm] 11.50 (s, 1H), 7.56 (t, J = 1.9, 1H), 7.41 (d, J = 1.9, 2H), 7.24 (d, J = 1.1, 1H), 7.12 (dd, J = 8.0, 1.1, 1H), 7.01 (d, J = 8.0, 1H), 5.09 (d, J = 4.0, 1H), 5.07 (s, 2H), 4.74-4.70 (m, 1H), 3.50 3.30 (m, 8H), 2.49-2.38 (m, 2H) | | |

### Pharmakologische Daten

### Autotaxin-Inhibierung (Enzym Test)

**Tabelle 1**

| Verbindung Nr. | IC50 |
|---|---|
| "11" | B |
| "13" | B |
| "15" | |
| "17" | B |
| "21" | C |
| "24" | C |
| "27" | C |
| "32" | |
| "33" | C |
| "39" | B |
| "A1" | B |
| "A2" | C |
| "A3" | C |
| "A4" | |
| "A5" | |
| "A6" | |
| "A7" | |
| "A8" | C |
| "A9" | |
| "A10" | C |
| "A11" | |
| "A12" | |
| "A13" | C |
| "A14" | C |
| "A15" | |
| "A16" | C |
| "A17" | C |
| "A18" | C |

| | |
|---|---|
| IC50: <100 nM = A 100 nM - 1 µM = B > 1 µM= C | |

### Beispiel A: Autotaxin Test (Enzym Test)

### Testbeschreibung

Die Autotaxin Aktivität wird indirekt mit dem Amplex Red Reagenz gemessen. Hierbei wird Amplex Red als fluorgenischem Indikatior für das entstandene H₂O₂ gemessen. Im Detail setzt Autotaxin das Substrat Lysophosphatidylcholin (LPC) zu Phosphocholin und Lysophosphatidylsäure (LPS) um. Nach dieser Umsetzung wird das Phosphocholin mit alkalischer Phosphatase zu inorganischem Phosphat und Cholin ungesetzt. Im nächsten Schritt wird Cholin durch Choline-Oxidase zu Betain oxidiert, wobei H₂O₂ entsteht. H₂O₂ reagiert in Gegenwart von Peroxidase (Horseradish peroxidase) mit dem Amplex Red Reagenz in eine 1:1 Stöchiometrie und bildet das hochfluoreszente Resorufin. Die Fluoreszenz wird in einem reaktionsabhängigen kinetischen Modus gemessen, damit dass fluoreszente Signale möglicher anderer fluoreszenter Stoffe, die nicht an der Reaktion beteiligt sind, herauskorrigiert werden kann.

### Testausführung

1,5 µl einer Standardlösung oder der Testsubstanzen (Substanzen mit dem Namen A(n)) in individuellen Konzentrationen gelöst in 20mM Hepes pH 7.2 mit maximal 7.7% DMSO werden zusammen mit 10 µl (16 ng) hochgereinigten recombinanten Autotaxin in einer schwarzen mit 384 Vertiefungen versehenen Mikrotiterplatte für 30 min bei 22°C vorinkubiert. Danach wird die Reaktion durch Zugabe von 5µl L-α-Lysophosphatidylcholin (LPC) gestartet, wobei die Endkonzentration von LPC 75 µM beträgt. Die Mischung wird 90 min. bei 37°C inkubiert. Nach der Inkubation wird Amplex Red Reagenz, Peroxidase (Horseradish peroxidase) und Cholin-Oxidase hinzugefügt und sofort die Fluoreszenz bei 612 nm bei einer Anregung von 485 nm in einem "Tecan Ultra multimode" Lesegerät gemessen. Die Aktivität von Autotaxin wird indirekt über den Nachweis des anfallenden H₂O₂ errechnet.

### Material:

| | |
|---|---|
| Microtiterplatte: | PS-Microplate, 384 Vertiefungen, kleines Volumen, schwarz Corning, Cat#3677 |
| Protein: | Recombinantes Autotaxin (Baculovirale Hi5 Expression) |
| Substrat: | L-α-Lysophosphatidylcholin (Hühnerei)); Avanti Polar Lipids # 830071P |
| Standard: | C14 LPA, Avanti Polar Lipids, Cat# 857120P |
| Nachweis Reagenz: | Amplex Red Reagenz ; Invitrogen # A12222; gelöst in 1.923 ml of DMSO Peroxidase Type VI-A (horseradish) von Sigma # P6782; gelöst in 7,45 ml Test Puffer, Choline-Oxidase ; Sigma # C5896; gelöst in 2,47 ml Test Puffer |
| Nachweis Reagenz Mix: | 1:100 Verdünnung von Amplex Red Regenzt in Test Puffer |
| Test Puffer: | 200 mM Tris-HCl, Merck, Cat # 1.08219, pH 7.9, 0.1 % BSA, lipidfrei, Roche Cat#775835 |

Die nachfolgenden Beispiele betreffen Arzneimittel:

### Beispiel B: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel C: Suppositorien

Man schmilzt ein Gemisch von 20 g eines Wirkstoffes der Formel I mit 100 g Sojalecithin und 1400 g Kakaobutter, gießt in Formen und lässt erkalten. Jedes Suppositorium enthält 20 mg Wirkstoff.

### Beispiel D: Lösung

Man bereitet eine Lösung aus 1 g eines Wirkstoffes der Formel I, 9,38 g NaH₂PO₄ 2 H₂O, 28,48 g Na₂HPO₄ 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel E: Salbe

Man mischt 500 mg eines Wirkstoffes der Formel I mit 99,5 g Vaseline unter aseptischen Bedingungen.

### Beispiel F: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel G: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel H: Kapseln

2 kg Wirkstoff der Formel I werden in üblicher Weise in Hartgelatinekapseln gefüllt, so dass jede Kapsel 20 mg des Wirkstoffs enthält.

### Bespiel I: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 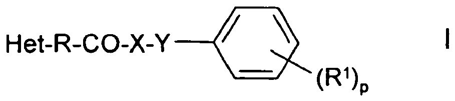 worin
R¹ H, A, Hal, OR³, N(R³)₂, N=CR³N(R³)₂, SR³, NO₂, CN, COOR³, CON(R³)₂, NR³COA, NR³SO₂A, SO₂N(R³)₂, S(O)ₘA, -[C(R³)₂]ₙN(R³)₂, O[C(R³)₂]ₚN(R³)₂, S[C(R³)₂]ₙN(R³)₂, -NR³[C(R³)₂]ₙN(R³)₂, NHCON(R³)₂, CON(R³)₂, CONR³[C(R³)₂]ₙN(R³)₂ oder COA,
R³ H oder A,
X O, NH oder CH₂,
Y CH₂, CH₂O oder fehlt,
R 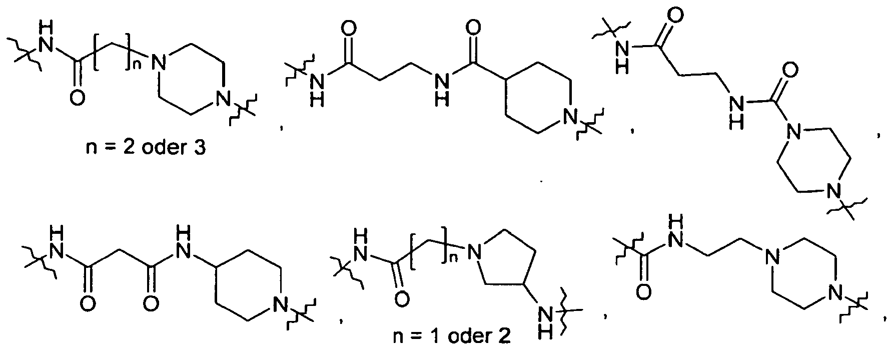 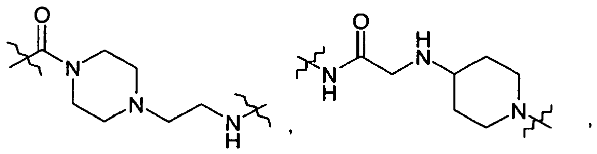 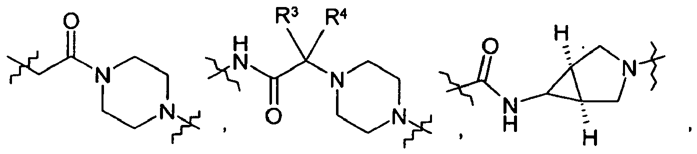 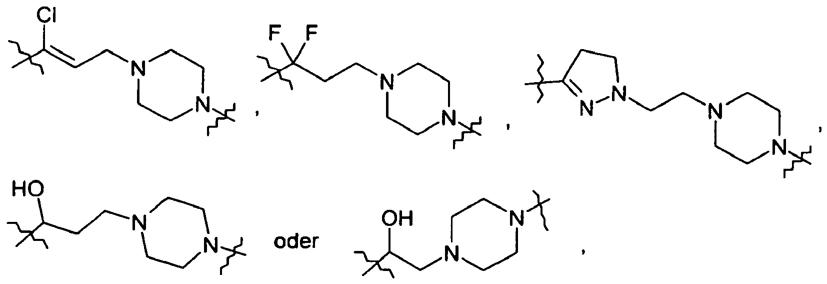
R⁴ H, A oder Phenyl,
Het 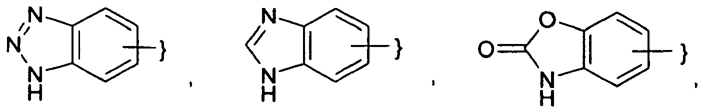 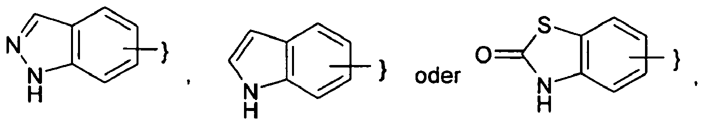
A unverzweigtes oder verzweigtes Alkyl mit 1-10 C-Atomen, worin 1-7 H-Atome durch OH, F, Cl und/oder Br ersetzt sein können, und/oder worin eine oder zwei CH₂-Gruppen durch O, NH und/oder S ersetzt sein können, oder cyclisches Alkyl mit 3-7 C-Atomen,
Hal F, Cl, Br oder I,
n 0, 1, 2 oder 3,
m 0, 1 oder 2,
p 0, 1, 2, 3, 4 oder 5,
bedeuten,
sowie ihre pharmazeutisch verwendbaren Salze und Stereoisomere,
einschließlich deren Mischungen in allen Verhältnissen.

2. Verbindungen nach Anspruch 1, worin
R¹ Hal bedeutet,
sowie ihre pharmazeutisch verwendbaren Salze und Stereoisomere,
einschließlich deren Mischungen in allen Verhältnissen.

3. Verbindungen nach Anspruch 1 oder 2, worin
X O oder CH₂,
bedeutet,
sowie ihre pharmazeutisch verwendbaren Salze und Stereoisomere, einschließlich deren Mischungen in allen Verhältnissen.

4. Verbindungen nach einem oder mehreren der Ansprüche 1-3, worin
Y CH₂ oder CH₂O
bedeutet,
sowie ihre pharmazeutisch verwendbaren Salze und Stereoisomere,
einschließlich deren Mischungen in allen Verhältnissen.

5. Verbindungen nach einem oder mehreren der Ansprüche 1-4, worin
A unverzweigtes oder verzweigtes Alkyl mit 1-10 C-Atomen, worin 1-7 H-Atome F und/oder Cl ersetzt sein können,
bedeutet,
sowie ihre pharmazeutisch verwendbaren Salze und Stereoisomere,
einschließlich deren Mischungen in allen Verhältnissen.

6. Verbindungen nach einem oder mehreren der Ansprüche 1-5, worin
p 1, 2 oder 3
bedeutet,
sowie ihre pharmazeutisch verwendbaren Salze und Stereoisomere,
einschließlich deren Mischungen in allen Verhältnissen.

7. Verbindungen nach einem oder mehreren der Ansprüche 1-6, worin
R¹ Hal,
X O oder CH₂,
Y CH₂ oder CH₂O,
R 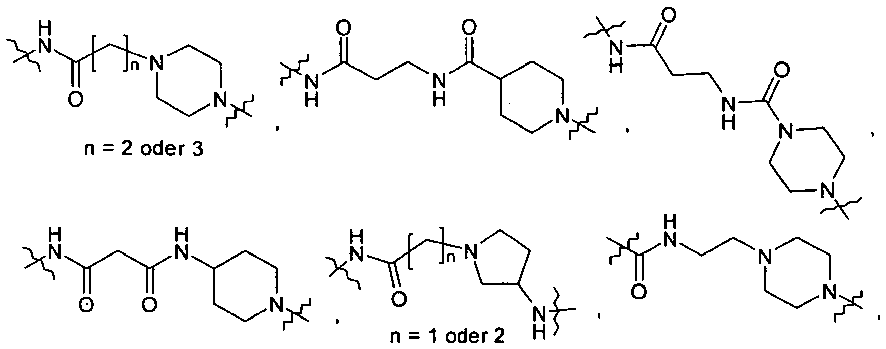 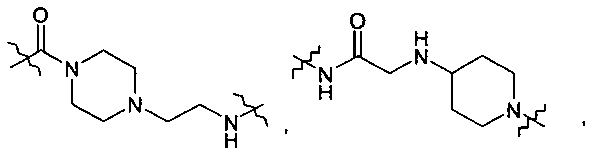 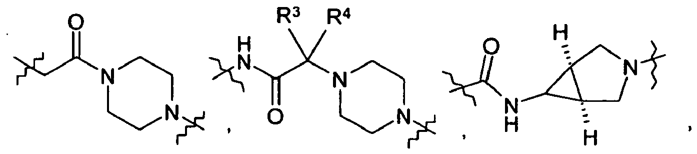 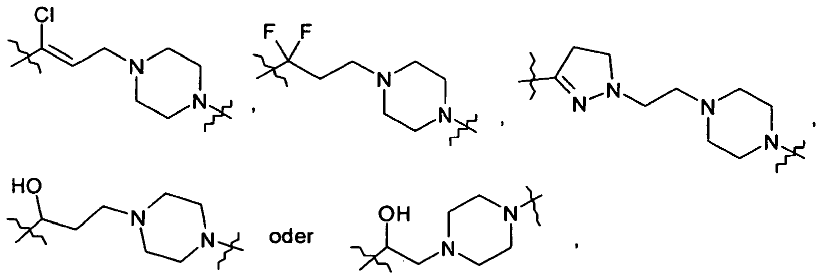 Het 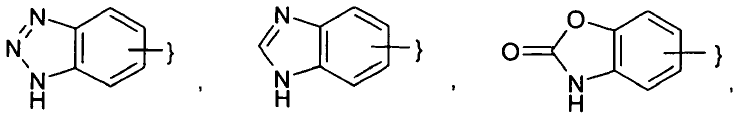 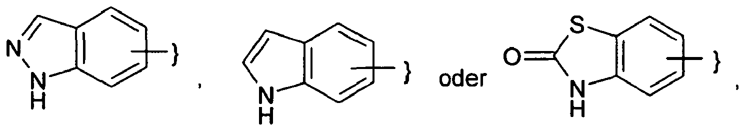
A unverzweigtes oder verzweigtes Alkyl mit 1-10 C-Atomen, worin 1-7 H-Atome F und/oder Cl ersetzt sein können,
Hal F, Cl, Br oder I,
p 1, 2 oder 3,
bedeuten,
sowie ihre pharmazeutisch verwendbaren Salze und Stereoisomere,
einschließlich deren Mischungen in allen Verhältnissen.

8. Verbindungen nach Anspruch 1 ausgewählt aus der Gruppe
| Nr. | Name und/oder Struktur |
|---|---|
| "11" | 4-{[(2-Oxo-2,3-dihydro-benzoxazol-6-ylcarbamoyl)-methyl]-amino}-piperidin-1-carbonsäure-3,5-dichlor-benzylester |
| "13" | 4-[3-Hydroxy-3-(2-oxo-2,3-dihydro-benzoxazol-6-yl)-propyl]-piperazin-1-carbonsäure-3,5-dichlor-benzylester |
| "15" | 4-[(Z)-3-Chlor-3-(2-oxo-2,3-dihydro-benzoxazol-6-yl)-allyl]-piperazin-1-carbonsäure-4-chlor-benzylester |
| "17" | 4-[3,3-Difluor-3-(2-oxo-2,3-dihydro-benzoxazol-6-yl)-propyl]-piperazin-1-carbonsäure-4-chlor-benzylester |
| "21" | 4-{2-[3-(2-Oxo-2,3-dihydro-benzoxazol-6-yl)-4,5-dihydro-pyrazol-1-yl]-ethyl}-piperazin-1-carbonsäure-3,5-dichlor-benzylester |
| "24" | 4-(2-1H-Benzotriazol-5-yl-acetyl)-piperazin-1-carbonsäure-4-chlor-benzylester |
| | |
| "32" | 4-[2-(1H-Benzotriazol-5-ylcarbamoyl)-ethylcarbamoyl]-piperidin-1-carbonsäure-4-chlor-benzylester |
| "33" | 4-[2-(1H-Benzotriazol-5-ylcarbamoyl)-ethylcarbamoyl]-piperazin-1-carbonsäure-4-chlor-benzylester |
| "39" | 4-[2-(1H-Benzotriazol-5-ylcarbamoyl)-acetylamino]-piperidin-1-carbonsäure-4-chlor-benzylester |
| "A1" | 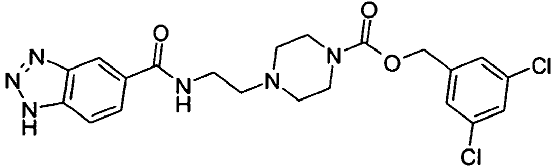 |
| | 4-{2-[(1H-Benzotriazol-5-carbonyl)-amino]-ethyl}-piperazin-1-carbonsäure-3,5-dichlor-benzylester |
| "A2" | 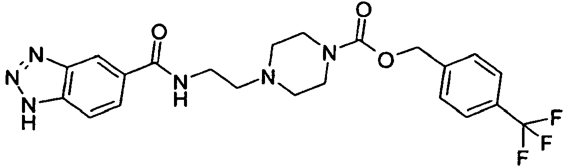 |
| | 4-{2-[(1H-Benzotriazol-5-carbonyl)-amino]-ethyl}-piperazin-1-carbonsäure-4-trifluormethyl-benzylester |
| "A3" | 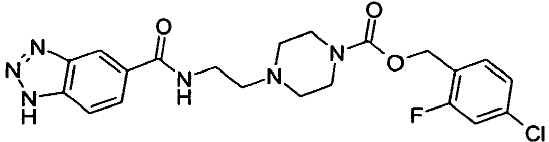 |
| | 4-{2-[(1H-Benzotriazol-5-carbonyl)-amino]-ethyl}-piperazin-1-carbonsäure-4-chlor-2-fluor-benzylester |
| "A4" | 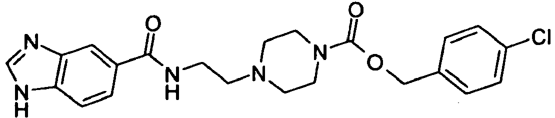 |
| | 4-{2-[(1H-Benzimidazol-5-carbonyl)-amino]-ethyl}-piperazin-1-carbonsäure-4-chlor-benzylester |
| "A5" | 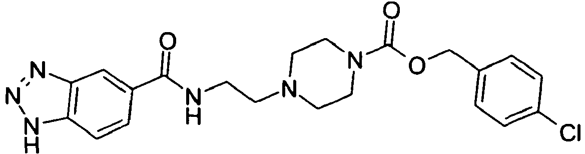 |
| | 4-{2-[(1H-Benzotriazol-5-carbonyl)-amino]-ethyl}-piperazin-1-carbonsäure-4-chlor-benzylester |
| "A6" | 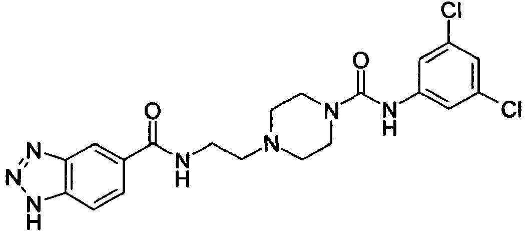 |
| | 1H-Benzotriazol-5-carbonsäure-{2-[4-(3,5-dichlor-phenylcarbamoyl)-piperazin-1-yl]-ethyl}-amid |
| "A7" | 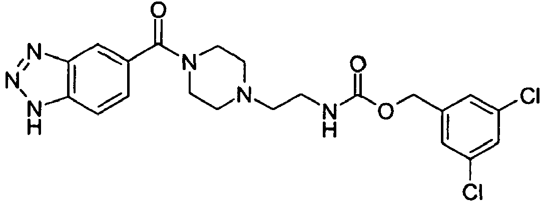 |
| | {2-[4-(1H-Benzotriazol-5-carbonyl)-piperazin-1-yl]-ethyl}-carbaminsäure-3,5-dichlor-benzylester |
| "A8" | 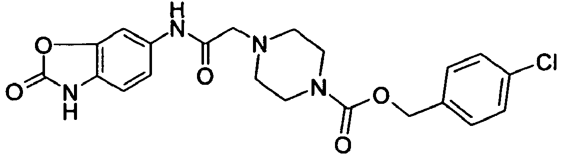 |
| | 4-[(2-Oxo-2,3-dihydro-benzoxazol-6-ylcarbamoyl)-methyl]-piperazin-1-carbonsäure-4-chlor-benzylester |
| "A9" | 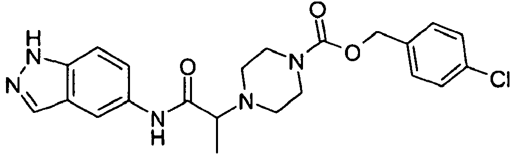 |
| | 4-[1-(1H-Indazol-5-ylcarbamoyl)-ethyl]-piperazin-1-carbonsäure-4-chlor-benzylester |
| "A10" | 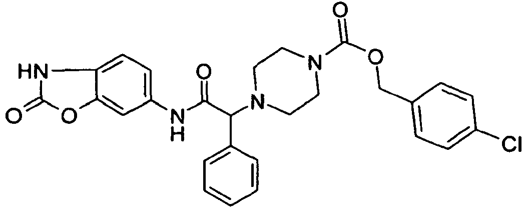 |
| | 4-[(2-Oxo-2,3-dihydro-benzoxazol-6-ylcarbamoyl)-phenyl-methyl]-piperazin-1-carbonsäure-4-chlor-benzylester |
| "A11" | 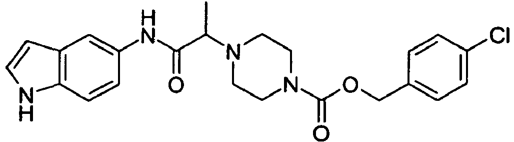 |
| | 4-[1-(1H-Indol-5-ylcarbamoyl)-ethyl]-piperazin-1-carbonsäure-4-chlor-benzylester |
| "A12" | 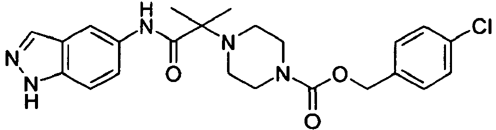 |
| | 4-[1-(1H-Indazol-5-ylcarbamoyl)-1-methyl-ethyl]-piperazin-1-carbonsäure-4-chlor-benzylester |
| "A13" | 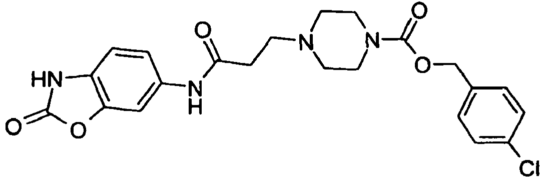 |
| | 4-[2-(2-Oxo-2,3-dihydro-benzoxazol-6-ylcarbamoyl)-ethyl]-piperazin-1-carbonsäure-4-chlor-benzylester |
| "A14" | 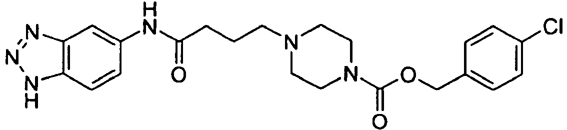 |
| | 4-[3-(1H-Benzotriazol-5-ylcarbamoyl)-propyl]-piperazin-1-carbonsäure-4-chlor-benzylester |
| "A15" | 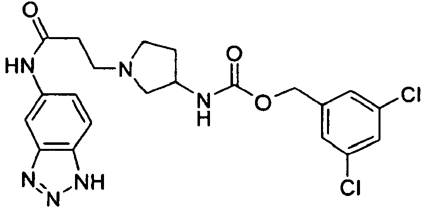 |
| | {1-[2-(1H-Benzotriazol-5-ylcarbamoyl)-ethyl]-pyrrolidin-3-yl}-carbaminsäure-3,5-dichlor-benzylester |
| "A16" | 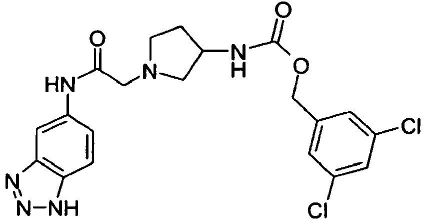 |
| | {1-[(1H-Benzotriazol-5-ylcarbamoyl)-methyl]-pyrrolidin-3-yl}-crbaminsäure-3,5-dichlor-benzylester |
| "A17" | 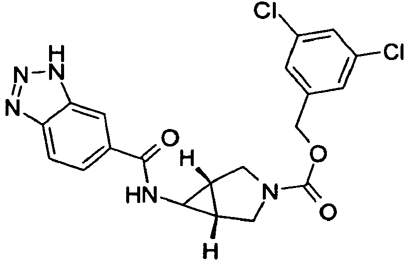 |
| | (1R,5S)-6-[(3H-Benzotriazole-5-carbonyl)-amino]-3-aza-bicyclo[3.1.0]hexan-3-carbonsäure-3,5-dichlor-benzylester |
| "A18" | 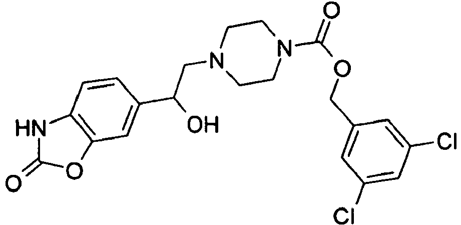 |
| | 4-[2-Hydroxy-2-(2-oxo-2,3-dihydro-benzoxazol-6-yl)-ethyl]-piperazin-1-carbonsäure-3,5-dichlor-benzylester |
sowie ihre pharmazeutisch verwendbaren Salze und Stereoisomere, einschließlich deren Mischungen in allen Verhältnissen.

9. Verfahren zur Herstellung von Verbindungen der Formel I sowie ihrer pharmazeutisch verwendbaren Salze und Stereoisomeren, **dadurch gekennzeichnet, dass** man
a) zur Herstellung von Verbindungen der Formel I, worin
R 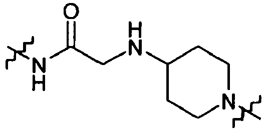 bedeutet,
eine Verbindung der Formel II
Het-NH-CO-CH₂-L II
worin
Het die in Anspruch 1 angegebene Bedeutung hat,
und L Cl oder Br bedeutet,
mit einer Verbindung der Formel III 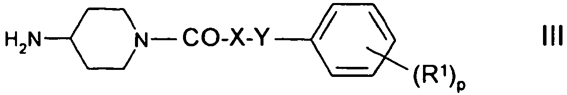 worin
X, Y, R¹ und p die in Anspruch 1 angegebenen Bedeutungen haben,
umsetzt,
oder
b) zur Herstellung von Verbindungen der Formel I, worin
R 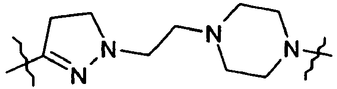 bedeutet,
eine Verbindung der Formel IV 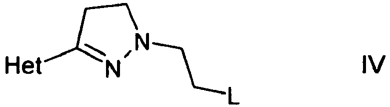 worin
Het die in Anspruch 1 angegebene Bedeutung hat,
und L Cl oder Br bedeutet,
mit einer Verbindung der Formel V 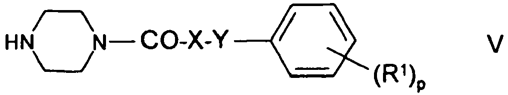 worin
X, Y, R¹ und p die in Anspruch 1 angegebenen
Bedeutungen haben,
umsetzt,
oder
c) zur Herstellung von Verbindungen der Formel I, worin
R 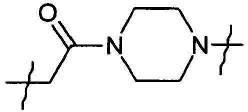 bedeutet,
eine Verbindung der Formel VI
Het-CH₂-CO-L VI
worin
Het die in Anspruch 1 angegebene Bedeutung hat,
und L Cl, Br, I oder eine freie oder reaktionsfähig funktionell abgewandelte OH-Gruppe bedeutet,
mit einer Verbindung der Formel V
umsetzt,
oder
d) zur Herstellung von Verbindungen der Formel I, worin
R 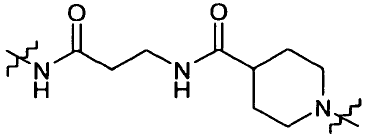 bedeutet,
eine Verbindung der Formel VII 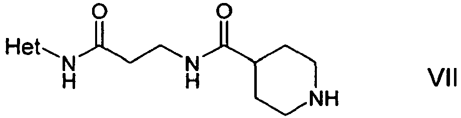 worin
Het die in Anspruch 1 angegebene Bedeutung hat,
mit einer Verbindung der Formel VIII 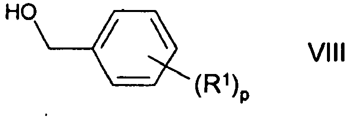 worin
R¹ und p die in Anspruch 1 angegebenen Bedeutungen haben,
und einer Verbindung ausgewählt aus der Gruppe Carbonyldiimidazol, Phosgen, Diphosgen, Triphosgen
umsetzt,
oder
e) zur Herstellung von Verbindungen der Formel I, worin
R 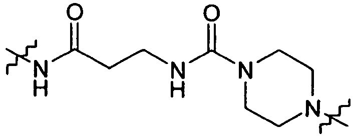 bedeutet,
eine Verbindung der Formel IX 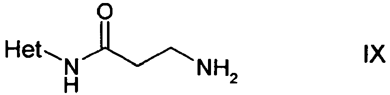 worin
Het die in Anspruch 1 angegebene Bedeutung hat,
mit einer Verbindung der Formel V
und einer Verbindung ausgewählt aus der Gruppe Carbonyldiimidazol, Phosgen, Diphosgen, Triphosgen,
umsetzt,
oder
f) zur Herstellung von Verbindungen der Formel I, worin
R 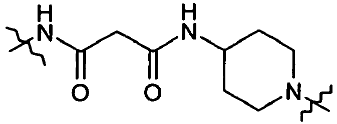 bedeutet,
eine Verbindung der Formel X
Het-NH₂ X
worin
Het die in Anspruch 1 angegebene Bedeutung hat,
mit einer Verbindung der Formel XI 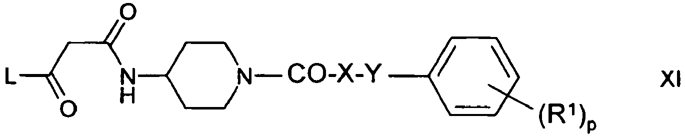 worin
X, Y, R¹, p die in Anspruch 1 angegebenen Bedeutungen haben,
und L Cl, Br, I oder eine freie oder reaktionsfähig funktionell abgewandelte OH-Gruppe bedeutet,
umsetzt,
und/oder eine Base oder Säure der Formel I in eines ihrer Salze umwandelt.

10. Arzneimittel, enthaltend mindestens eine Verbindung der Formel I nach Anspruch 1 bis 8 und/oder ihre pharmazeutisch verwendbaren Salze und Stereoisomeren, einschließlich deren Mischungen in allen Verhältnissen, sowie gegebenenfalls Träger- und/oder Hilfsstoffe.

11. Verwendung von Verbindungen nach Anspruch 1 bis 8, zur Herstellung eines Arzneimittels zur Behandlung und Prophylaxe von Krebskrankheiten.

12. Verwendung nach Anspruch 11, wobei die Krebskrankheiten mit einem Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge einhergehen.

13. Verwendung nach Anspruch 12, wobei der Tumor aus der Gruppe Monozytenleukämie, Lungenadenokarzinom, kleinzellige Lungenkarzinome, Bauchspeicheldrüsenkrebs, Ovarialkarzinom, Glioblastome und Brustkarzinom und Kolokarzinom stammt.

14. Verwendung nach Anspruch 13, wobei die zu behandelnde Krankheit ein Tumor des Blut- und Immunsystems ist.

15. Verwendung nach Anspruch 14, wobei der Tumor aus der Gruppe der akuten myelotischen Leukämie, der chronischen myelotischen Leukämie, akuten lymphatischen Leukämie und/oder chronischen lymphatischen Leukämie stammt.

16. Verwendung von Verbindungen der Formel I gemäß Anspruch 1 bis 8 und/oder ihrer physiologisch unbedenklichen Salze und Solvate zur Herstellung eines Arzneimittels zur Behandlung von Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

17. Die Verbindung 6-(2-{4-[3-(4-Chlor-phenoxy)-propionyl]-piperazin-1-yl}-acetyl)-3H-benzoxazol-2-on sowie seine pharmazeutisch verwendbaren Salze und Stereoisomeren, einschließlich deren Mischungen in allen Verhältnissen.

## Claims

1. Compounds of the formula I 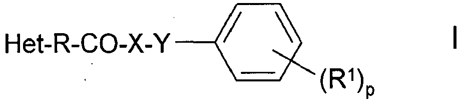 in which
R¹ denotes H, A, Hal, OR³, N(R³)₂, N=CR³N(R³)₂, SR³, NO₂, CN, COOR³, CON(R³)₂, NR³COA, NR³SO₂A, SO₂N(R³)₂, S(O)ₘA, -[C(R³)₂]ₙN(R³)₂, O[C(R³)₂]ₚN(R³)₂, S[C(R³)₂]ₙN(R³)₂, -NR³[C(R³)₂]ₙN(R³)₂, NHCON(R³)₂, CON(R³)₂, CONR³[C(R³)₂]ₙN(R³)₂ or COA,
R³ denotes H or A,
X denotes O, NH or CH₂,
Y denotes CH₂, CH₂O or is absent,
R denotes 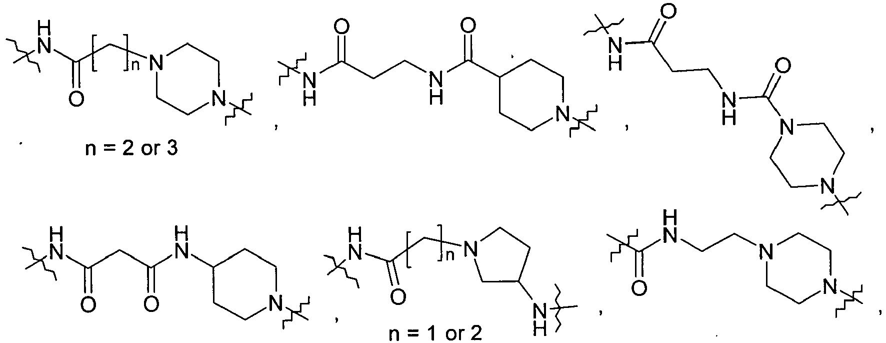 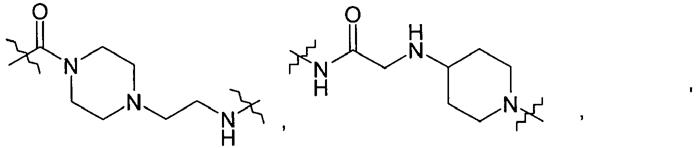 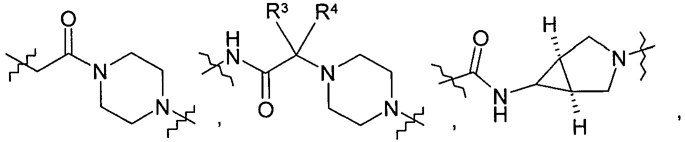 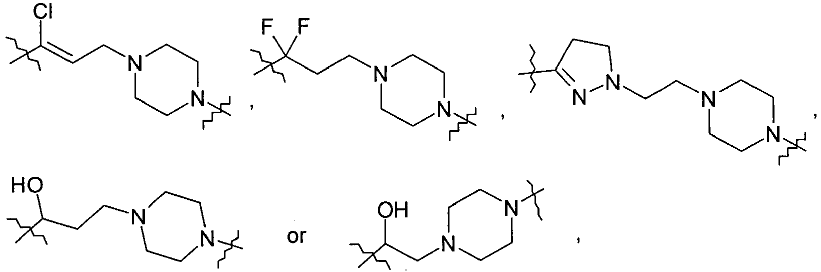
R⁴ denotes H, A or phenyl,
Het denotes 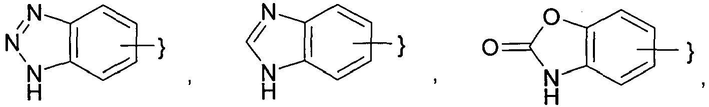 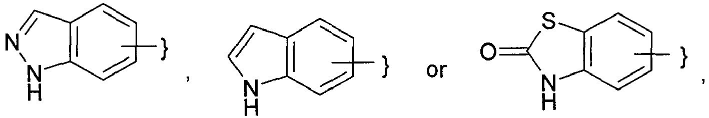
A denotes unbranched or branched alkyl having 1-10 C atoms, in which 1-7 H atoms may be replaced by OH, F, Cl and/or Br, and/or in which one or two CH₂ groups may be replaced by O, NH and/or S,
or
cyclic alkyl having 3-7 C atoms,
Hal denotes F, Cl, Br or I,
n denotes 0, 1, 2 or 3,
m denotes 0, 1 or 2,
p denotes 0, 1, 2, 3, 4 or 5,
and pharmaceutically usable salts and stereoisomers thereof, including mixtures thereof in all ratios.

2. Compounds according to Claim 1 in which
R¹ denotes Hal,
and pharmaceutically usable salts and stereoisomers thereof, including mixtures thereof in all ratios.

3. Compounds according to Claim 1 or 2 in which
X denotes O or CH₂,
and pharmaceutically usable salts and stereoisomers thereof, including mixtures thereof in all ratios.

4. Compounds according to one or more of Claims 1-3 in which
Y denotes CH₂ or CH₂O,
and pharmaceutically usable salts and stereoisomers thereof, including mixtures thereof in all ratios.

5. Compounds according to one or more of Claims 1-4 in which
A denotes unbranched or branched alkyl having 1-10 C atoms, in which 1-7 H atoms may be replaced by F and/or Cl,
and pharmaceutically usable salts and stereoisomers thereof, including mixtures thereof in all ratios.

6. Compounds according to one or more of Claims 1-5 in which
p denotes 1, 2 or 3,
and pharmaceutically usable salts and stereoisomers thereof, including mixtures thereof in all ratios.

7. Compounds according to one or more of Claims 1-6 in which
R¹ denotes Hal,
X denotes O or CH₂,
Y denotes CH₂ or CH₂O,
R denotes 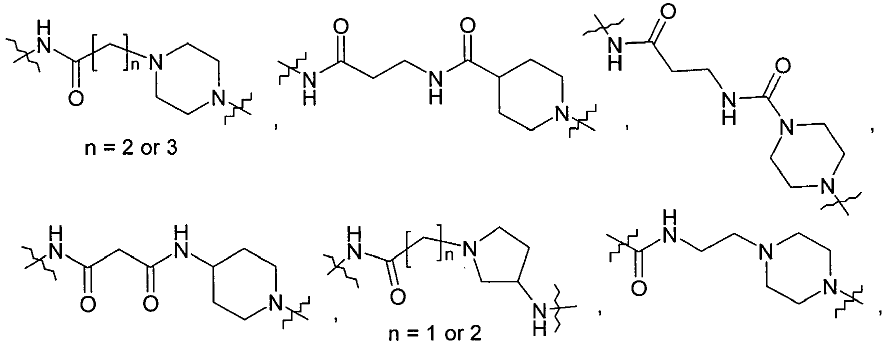 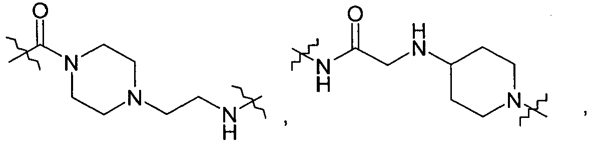 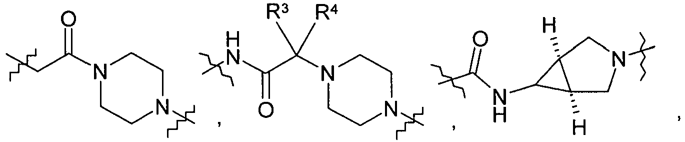 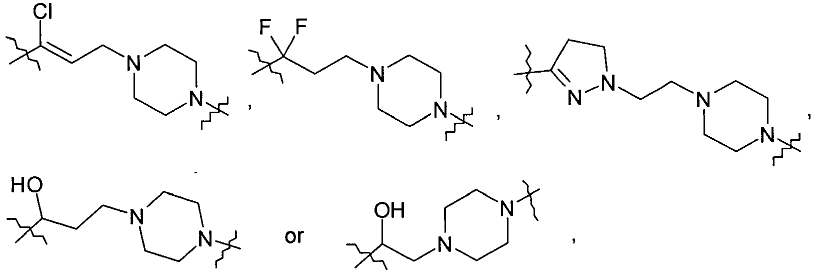
Het denotes 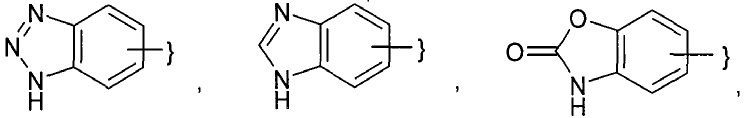 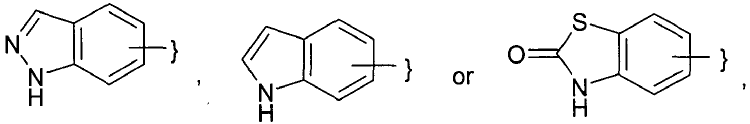
A denotes unbranched or branched alkyl having 1-10 C atoms, in which 1-7 H atoms may be replaced by F and/or Cl,
Hal denotes F, Cl, Br or I,
p denotes 1, 2 or 3,
and pharmaceutically usable salts and stereoisomers thereof, including mixtures thereof in all ratios.

8. Compounds according to Claim 1 selected from the group
| No. | Name and/or structure |
|---|---|
| "11" | 3,5-Dichlorobenzyl 4-{[(2-oxo-2,3-dihydrobenzoxazol-6-yl-carbamoyl)methyl]amino}piperidine-1-carboxylate |
| "13" | 3,5-Dichlorobenzyl 4-[3-hydroxy-3-(2-oxo-2,3-dihydrobenzoxazol-6-yl)propyl]piperazine-1-carboxylate |
| "15" | 4-Chlorobenzyl 4-[(Z)-3-chloro-3-(2-oxo-2, 3-d ihyd robenzoxazol-6-yl)allyl]piperazine-1-carboxylate |
| "17" | 4-Chlorobenzyl 4-[3,3-difluoro-3-(2-oxo-2,3-dihydrobenzoxazol-6-yl)propyl]piperazine-1-carboxylate |
| "21" | 3,5-Dichlorobenzyl 4-{2-[3-(2-oxo-2,3-dihydrobenzoxazol-6-yl)-4,5-dihydropyrazol-1-yl]ethyl}piperazine-1-carboxylate |
| "24" | 4-Chlorobenzyl 4-(2-1H-benzotriazol-5-ylacetyl)piperazine-1-carboxylate |
| "32" | 4-Chlorobenzyl 4-[2-(1 H-benzotriazol-5-yl-carbamoyl)ethylcarbamoyl]piperidine-1-carboxylate |
| "33" | 4-Chlorobenzyl 4-[2-(1H-benzotriazol-5-ylcarbamoyl)ethylcarbamoyl]piperazine-1-carboxylate |
| "39" | 4-Chlorobenzyl 4-[2-(1H-benzotriazol-5-ylcarbamoyl)acetyl-amino]piperidine-1-carboxylate |
| "A1" | 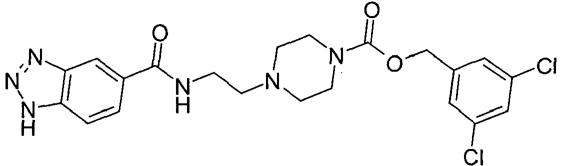 |
| | 3,5-Dichlorobenzyl 4-{2-[(1H-benzotriazole-5-carbonyl)-amino]ethyl}piperazine-1-carboxylate |
| "A2" | 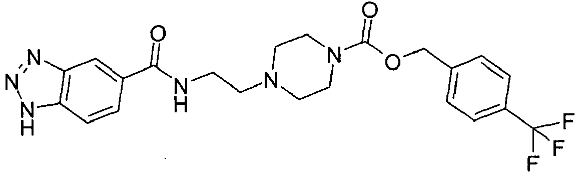 |
| | 4-Trifluoromethylbenzyl 4-{2-[(1H-benzotriazole-5-carbonyl)-amino]ethyl}piperazine-1-carboxylate |
| "A3" | 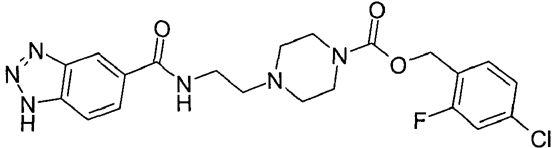 |
| | 4-Chloro-2-fluorobenzyl 4-{2-[(1H-benzotriazole-5-carbonyl)-amino]ethyl}piperazine-1-carboxylate |
| "A4" | 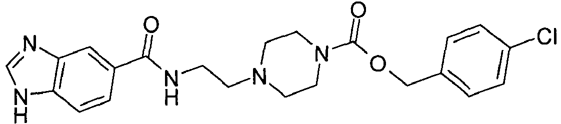 |
| | 4-Chlorobenzyl 4-{2-[(1H-benzimidazole-5-carbonyl)amino]-ethyl}piperazine-1-carboxylate |
| "A5" | 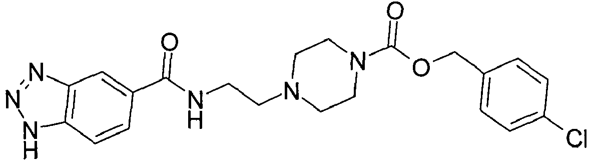 |
| | 4-Chlorobenzyl 4-{2-[(1H-benzotriazole-5-carbonyl)amino]-ethyl}piperazine-1-carboxylate |
| "A6" | 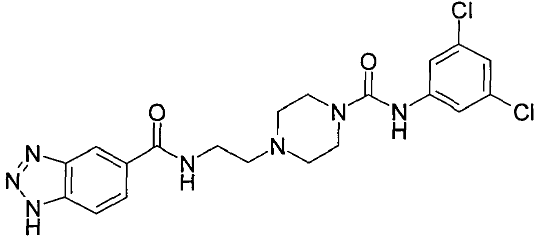 |
| | N-{2-[4-(3,5-Dichlorophenylcarbamoyl)piperazin-1-yl]ethyl}-1H-benzotriazole-5-carboxamide |
| "A7" | 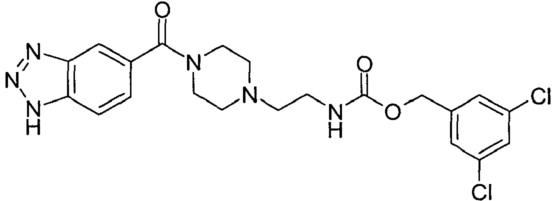 |
| | 3,5-Dichlorobenzyl {2-[4-(1H-benzotriazole-5-carbonyl)-piperazin-1-yl]ethyl}carbamate |
| "A8" | 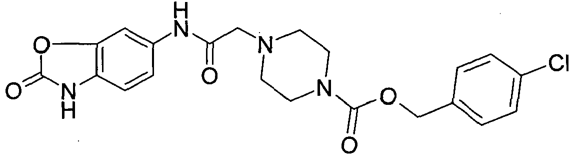 |
| | 4-Chlorobenzyl 4-[(2-oxo-2,3-dihydrobenzoxazol-6-yl-carbamoyl)methyl]piperazine-1-carboxylate |
| "A9" | 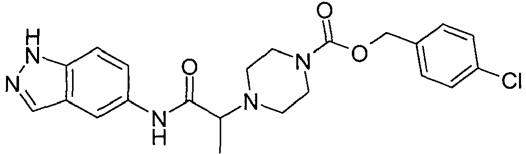 |
| | 4-Chlorobenzyl 4-[1-(1H-indazol-5-ylcarbamoyl)ethyl]-piperazine-1-carboxylate |
| "A10" | 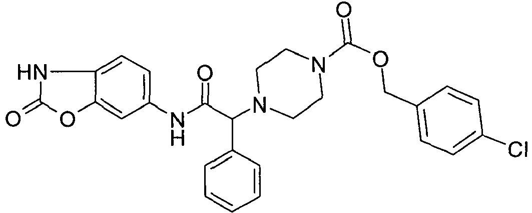 |
| | 4-Chlorobenzyl 4-[(2-oxo-2,3-dihydrobenzoxazol-6-yl-carbamoyl)phenylmethyl]piperazine-1-carboxylate |
| "A11" | 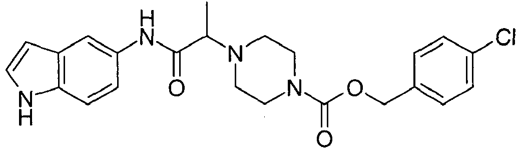 |
| | 4-Chlorobenzyl 4-[1-(1H-indol-5-ylcarbamoyl)ethyl]-piperazine-1-carboxylate |
| "A12" | 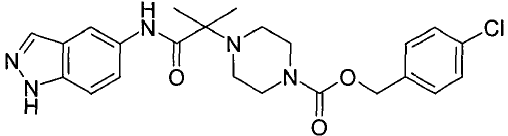 |
| | 4-Chlorobenzyl 4-[1-(1H-indazol-5-ylcarbamoyl)-1-methyl-ethyl]piperazine-1-carboxylate |
| "A13" | 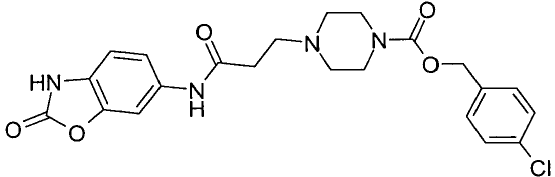 |
| | 4-Chlorobenzyl 4-[2-(2-oxo-2,3-dihydrobenzoxazol-6-yl-carbamoyl)ethyl]piperazine-1-carboxylate |
| "A14" | 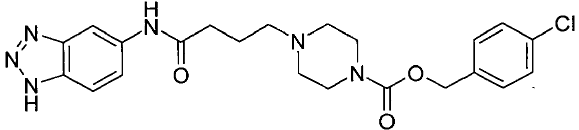 |
| | 4-Chlorobenzyl 4-[3-(1H-benzotriazol-5-ylcarbamoyl)propyl]-piperazine-1-carboxylate |
| "A15" | 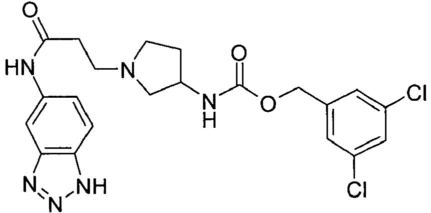 |
| | 3,5-Dichlorobenzyl {1-[2-(1H-benzotriazol-5-ylcarbamoyl)-ethyl]pyrrolidin-3-yl}carbamate |
| "A16" | 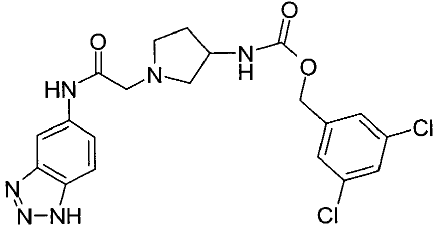 |
| | 3,5-Dichlorobenzyl {1-[(1H-benzotriazol-5-ylcarbamoyl)-methyl]pyrrolidin-3-yl}carbamate |
| "A17" | 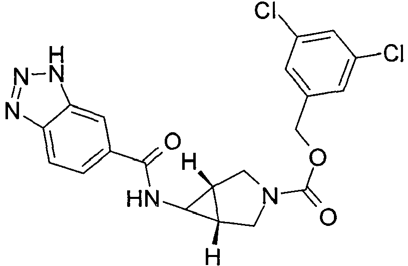 |
| | 3,5-Dichlorobenzyl (1R,5S)-6-[(3H-benzotriazole-5-carbonyl)amino]-3-azabicyclo[3.1.0]hexane-3-carboxylate |
| "A18" | 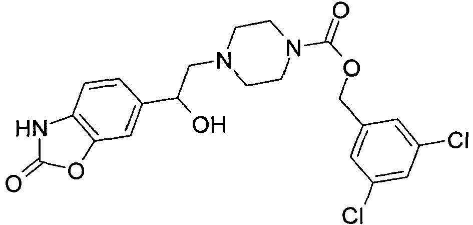 |
| | 3,5-Dichlorobenzyl 4-[2-hydroxy-2-(2-oxo-2,3-dihydrobenzoxazol-6-yl)ethyl]piperazine-1-carboxylate |
and pharmaceutically usable salts and stereoisomers thereof, including mixtures thereof in all ratios.

9. Process for the preparation of compounds of the formula I and pharmaceutically usable salts and stereoisomers thereof, **characterised in that**
a) for the preparation of compounds of the formula I in which
R denotes 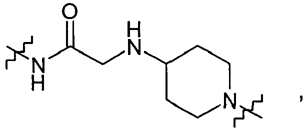 a compound of the formula II
Het-NH-CO-CH₂-L II
in which
Het has the meaning indicated in Claim 1,
and L denotes Cl or Br,
is reacted with a compound of the formula III 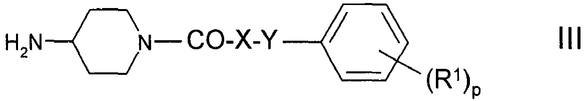 in which
X, Y, R¹ and p have the meanings indicated in Claim 1,
or
b) for the preparation of compounds of the formula I in which
R denotes 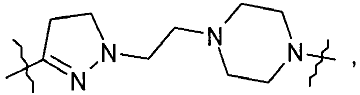 a compound of the formula IV 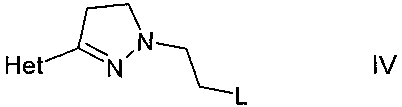 in which
Het has the meaning indicated in Claim 1,
and L denotes CI or Br,
is reacted with a compound of the formula V 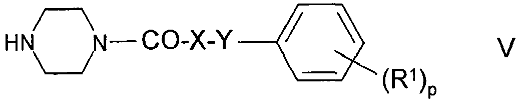 in which
X, Y, R¹ and p have the meanings indicated in Claim 1,
or
c) for the preparation of compounds of the formula I in which
R denotes 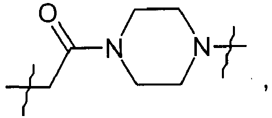 a compound of the formula VI
Het-CH₂-CO-L VI
in which
Het has the meaning indicated in Claim 1,
and L denotes Cl, Br, I or a free or reactively functionally modified OH group,
is reacted with a compound of the formula V,
or
d) for the preparation of compounds of the formula I in which
R denotes 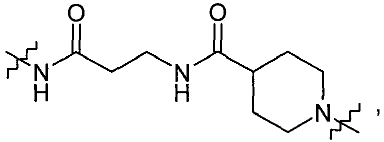 a compound of the formula VII 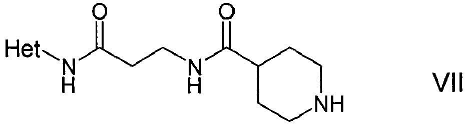 in which
Het has the meaning indicated in Claim 1,
is reacted with a compound of the formula VIII 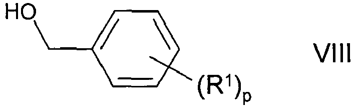 in which
R¹ and p have the meanings indicated in Claim 1,
and a compound selected from the group carbonyldiimidazole, phosgene, diphosgene, triphosgene,
or
e) for the preparation of compounds of the formula I in which
R denotes 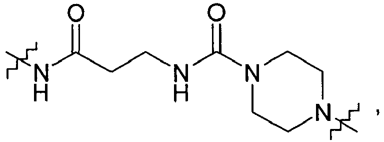 a compound of the formula IX 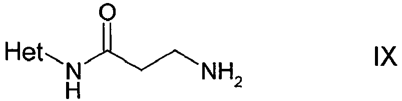 in which
Het has the meaning indicated in Claim 1,
is reacted with a compound of the formula V
and a compound selected from the group carbonyldiimidazole, phosgene, diphosgene, triphosgene,
or
f) for the preparation of compounds of the formula I in which
R denotes 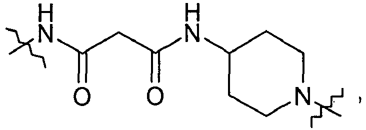 a compound of the formula X
Het-NH₂ X
in which
Het has the meaning indicated in Claim 1,
is reacted with a compound of the formula XI 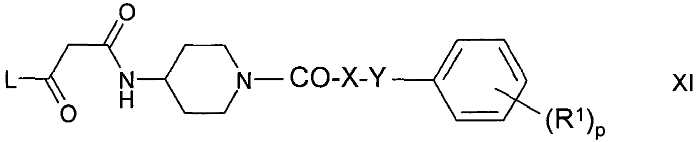 in which
X, Y, R¹, p have the meanings indicated in Claim 1,
and L denotes Cl, Br, I or a free or reactively functionally modified OH group,
and/or a base or acid of the formula I is converted into one of its salts.

10. Medicaments comprising at least one compound of the formula I according to Claims 1 to 8 and/or pharmaceutically usable salts and stereoisomers thereof, including mixtures thereof in all ratios, and optionally excipients and/or adjuvants.

11. Use of compounds according to Claims1 to 8 for the preparation of a medicament for the treatment and prophylaxis of cancer diseases.

12. Use according to Claim 11, where the cancer diseases are associated with a tumour from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

13. Use according to Claim 12, where the tumour originates from the group monocytic leukaemia, lung adenocarcinoma, small-cell lung carcinomas, pancreatic cancer, ovarian carcinoma, glioblastomas and breast carcinoma and colon carcinoma.

14. Use according to Claim 13, where the disease to be treated is a tumour of the blood and immune system.

15. Use according to Claim 14, where the tumour originates from the group of acute myeloid leukaemia, chronic myeloid leukaemia, acute lymphatic leukaemia and/or chronic lymphatic leukaemia.

16. Use of compounds of the formula I according to Claims 1 to 8 and/or physiologically acceptable salts and solvates thereof for the preparation of a medicament for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) other angiogenesis inhibitors.

17. The compound 6-(2-{4-[3-(4-chlorophenoxy)propionyl]piperazin-1-yl}-acetyl)-3H-benzoxazol-2-one and pharmaceutically usable salts and stereoisomers thereof, including mixtures thereof in all ratios.

## Revendications

1. Composés de formule I 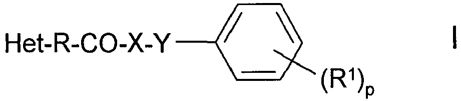 dans laquelle
R¹ désigne H, A, Hal, OR³, N(R³)₂, N=CR³N(R³)₂, SR³, NO₂, CN, COOR³, CON(R³)₂, NR³COA, NR³SO₂A, SO₂N(R³)₂, S(O)ₘA, -[C(R³)₂]ₙN(R³)₂, O[C(R³)₂]ₚN(R³)₂, S[C(R³)₂]ₙN(R³)₂, -NR³[C(R³)₂]ₙN(R³)₂, NHCON(R³)₂, CON(R³)₂, CONR³[C(R³)₂]ₙN(R³)₂ ou COA,
R³ désigne H ou A,
X désigne O, NH ou CH₂,
Y désigne CH₂, CH₂O ou est absent,
R désigne 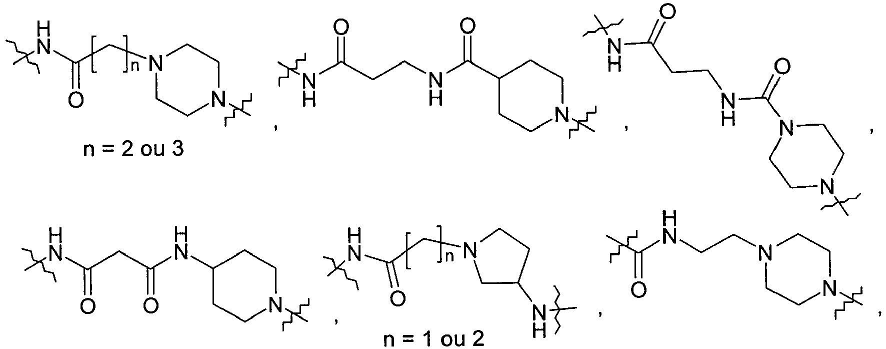 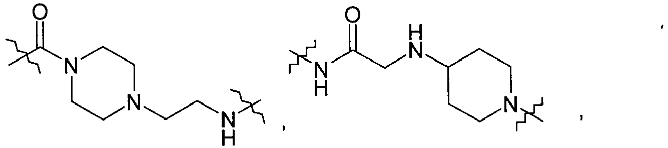 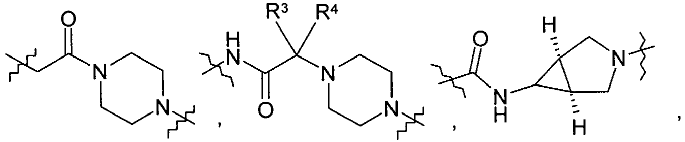 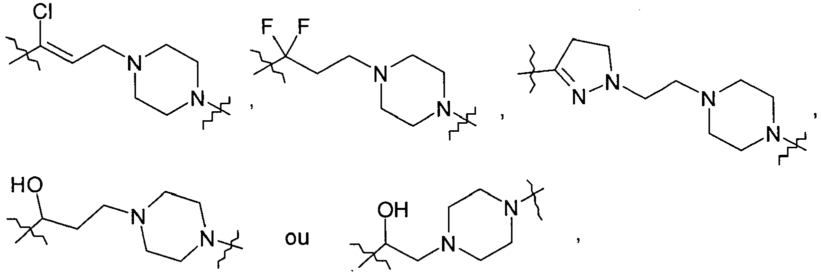
R⁴ désigne H, A ou phényle,
Hét désigne 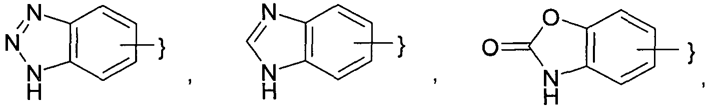 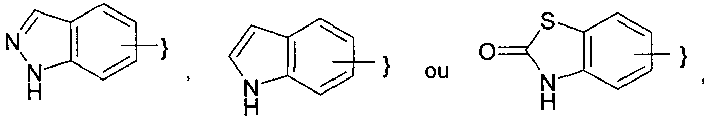
A désigne alkyle non ramifié ou ramifié ayant 1-10 atomes de C, où 1-7 atomes de H peuvent être remplacés par OH, F, CI et/ou Br, et/ou où un ou deux groupements CH₂ peuvent être remplacés par O, NH et/ou S, ou
alkyle cyclique ayant 3-7 atomes de C,
Hal désigne F, Cl, Br ou I,
n désigne 0, 1, 2 ou 3,
m désigne 0, 1 ou 2,
p désigne 0, 1, 2, 3, 4 ou 5,
et les sels et stéréoisomères pharmaceutiquement utilisables de ceux-ci, y compris des mélanges de ceux-ci en toutes proportions.

2. Composés selon la revendication 1, dans lesquels
R¹ désigne Hal,
et les sels et stéréoisomères pharmaceutiquement utilisables de ceux-ci, y compris des mélanges de ceux-ci en toutes proportions.

3. Composés selon la revendication 1 ou 2, dans lesquels
X désigne O ou CH₂,
et les sels et stéréoisomères pharmaceutiquement utilisables de ceux-ci, y compris des mélanges de ceux-ci en toutes proportions.

4. Composés selon l'une ou plusieurs parmi les revendications 1-3, dans lesquels
Y désigne CH₂ ou CH₂O,
et les sels et stéréoisomères pharmaceutiquement utilisables de ceux-ci, y compris des mélanges de ceux-ci en toutes proportions.

5. Composés selon l'une ou plusieurs parmi les revendications 1-4, dans lesquels
A désigne alkyle non ramifié ou ramifié ayant 1-10 atomes de C, où 1-7 atomes de H peuvent être remplacés par F et/ou Cl,
et les sels et stéréoisomères pharmaceutiquement utilisables de ceux-ci, y compris des mélanges de ceux-ci en toutes proportions.

6. Composés selon l'une ou plusieurs parmi les revendications 1-5, dans lesquels
p désigne 1, 2 ou 3,
et les sels et stéréoisomères pharmaceutiquement utilisables de ceux-ci, y compris des mélanges de ceux-ci en toutes proportions.

7. Composés selon l'une ou plusieurs parmi les revendications 1-6, dans lesquels
R¹ désigne Hal,
X désigne O ou CH₂,
Y désigne CH₂ ou CH₂O,
R désigne 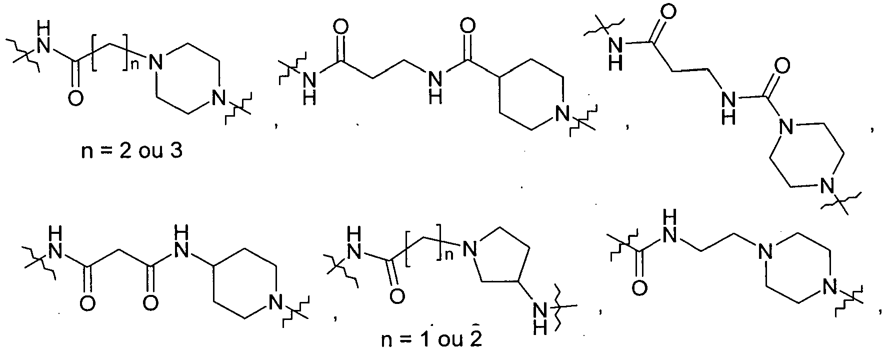 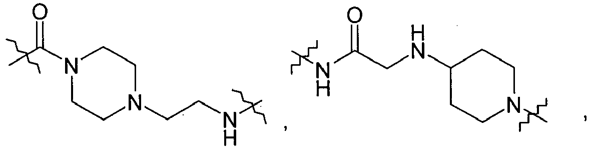 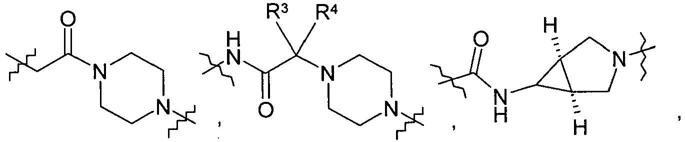 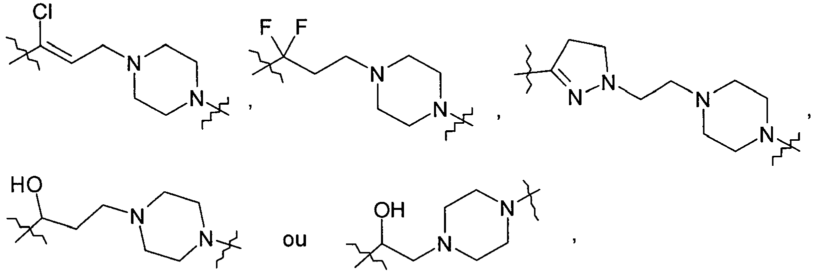
Hét désigne 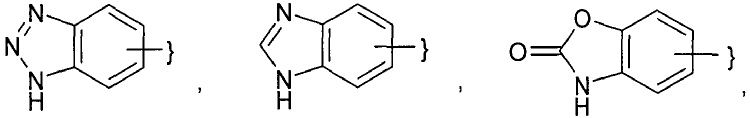 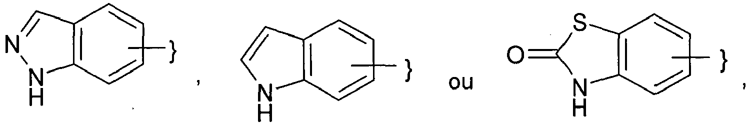
A désigne alkyle non ramifié ou ramifié ayant 1-10 atomes de C, où 1-7 atomes de H peuvent être remplacés par F et/ou Cl,
Hal désigne F, Cl, Br ou I,
p désigne 1, 2 ou 3,
et les sels et stéréoisomères pharmaceutiquement utilisables de ceux-ci, y compris des mélanges de ceux-ci en toutes proportions.

8. Composés selon la revendication 1, choisis dans le groupe constitué par
| n° | Nom et/ou structure |
|---|---|
| "11" | 4-{[(2-oxo-2,3-dihydrobenzoxazol-6-ylcarbamoyl)méthyl]-amino}pipéridine-1-carboxylate de 3,5-dichlorobenzyle |
| "13" | 4-[3-hydroxy-3-(2-oxo-2,3-dihydrobenzoxazol-6-yl)-propyl]pipérazine-1-carboxylate de 3,5-dichlorobenzyle |
| "15" | 4-[(Z)-3-chloro-3-(2-oxo-2,3-dihydrobenzoxazol-6-yl)-allyl]pipérazine-1-carboxylate de 4-chlorobenzyle |
| "17" | 4-[3,3-difluoro-3-(2-oxo-2,3-dihydrobenzoxazol-6-yl)-propyl]pipérazine-1-carboxylate de 4-chlorobenzyle |
| "21" | 4-{2-[3-(2-oxo-2,3-dihydrobenzoxazol-6-yl)-4,5-dihydropyrazol-1-yl]éthyl}pipérazine-1-carboxylate de 3,5-dichlorobenzyle |
| "24" | 4-(2-1 H-benzotriazol-5-ylacétyl)pipérazine-1-carboxylate de 4-chlorobenzyle |
| | |
| "32" | 4-[2-(1H-benzotriazol-5-ylcarbamoyl)éthylcarbamoyl]-pipéridine-1-carboxylate de 4-chlorobenzyle |
| "33" | 4-[2-(1 H-benzotriazol-5-ylcarbamoyl)éthylcarbamoyl]-pipérazine-1-carboxylate de 4-chlorobenzyle |
| "39" | 4-[2-(1H-benzotriazol-5-ylcarbamoyl)acétylamino]pipéridine-1-carboxylate de 4-chlorobenzyle |
| "A1" | 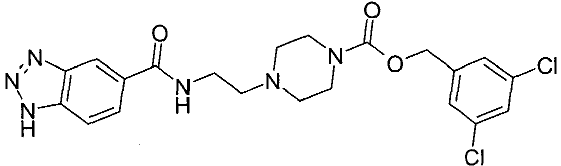 |
| | 4-{2-[(1H-benzotriazole-5-carbonyl)amino]éthyl}pipérazine-1-carboxylate de 3,5-dichlorobenzyle |
| "A2" | 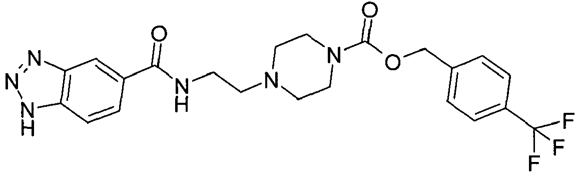 |
| | 4-{2-[(1H-benzotriazole-5-carbonyl)amino]éthyl}pipérazine-1-carboxylate de 4-trifluorométhylbenzyle |
| "A3" | 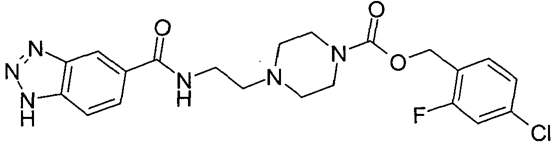 |
| | 4-{2-[(1H-benzotriazole-5-carbonyl)amino]éthyl}pipérazine-1-carboxylate de 4-chloro-2-fluorobenzyle |
| "A4" | 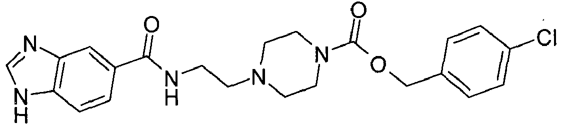 |
| | 4-{2-[(1H-benzimidazole-5-carbonyl)amino]éthyl}pipérazine-1-carboxylate de 4-chlorobenzyle |
| "A5" | 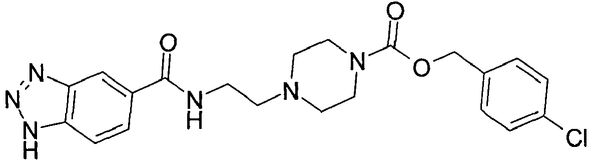 |
| | 4-{2-[(1H-benzotriazole-5-carbonyl)amino]éthyl}pipérazine-1-carboxylate de 4-chlorobenzyle |
| "A6" | 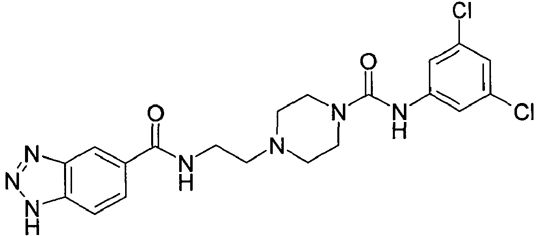 |
| | N-{2-[4-(3,5-dichlorophénylcarbamoyl)pipérazin-1-yl]éthyl}-1H-benzotriazole-5-carboxamide |
| "A7" | 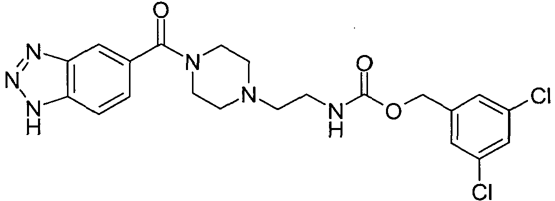 |
| | {2-[4-(1H-benzotriazole-5-carbonyl)pipérazin-1-yl]-éthyl}carbamate de 3,5-dichlorobenzyle |
| "A8" | 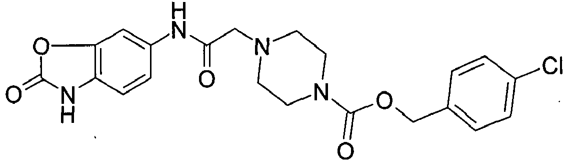 |
| | 4-[(2-oxo-2,3-dihydrobenzoxazol-6-ylcarbamoyl)méthyl]-pipérazine-1-carboxylate de 4-chlorobenzyle |
| "A9" | 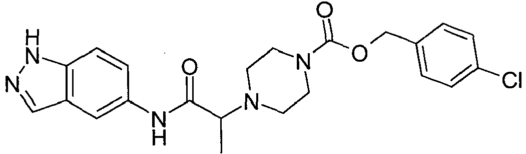 |
| | 4-[1-(1H-indazo)-5-ylcarbamoyl)éthyl]pipérazine-1-carboxylate de 4-chlorobenzyle |
| "A10" | 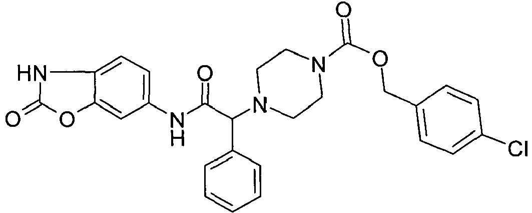 |
| | 4-[(2-oxo-2,3-dihydrobenzoxazol-6-ylcarbamoyl)-phénylméthyl]pipérazine-1-carboxylate de 4-chlorobenzyle |
| "A11 | 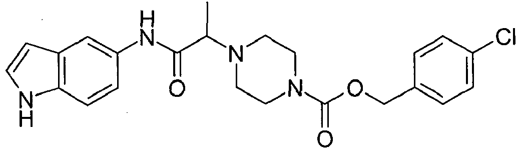 |
| | 4-[1-(1H-indol-5-ylcarbamoyl)éthyl]pipérazine-1-carboxylate de 4-chlorobenzyle |
| "A12" | 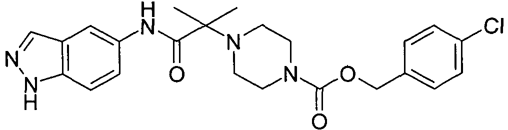 |
| | 4-[1-(1H-indazol-5-ylcarbamoyl)-1-méthyléthyl]pipérazine-1-carboxylate de 4-chlorobenzyle |
| "A13" | 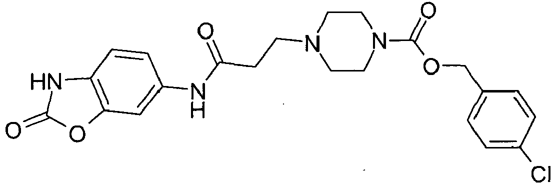 |
| | 4-[2-(2-oxo-2,3-dihydrobenzoxazol-6-ylcarbamoyl)éthyl]-pipérazine-1-carboxylate de 4-chlorobenzyle |
| "A14" | 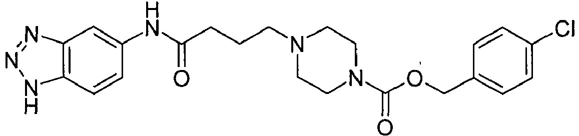 |
| | 4-[3-(1H-benzotriazol-5-ylcarbamoyl)propyl]pipérazine-1-carboxylate de 4-chlorobenzyle |
| "A15" | 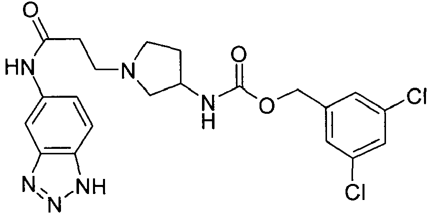 |
| | {1-[2-(1H-benzotriazol-5-ylcarbamoyl)éthyl]pyrrolidin-3-yl}-carbamate de 3,5-dichlorobenzyle |
| "A16" | 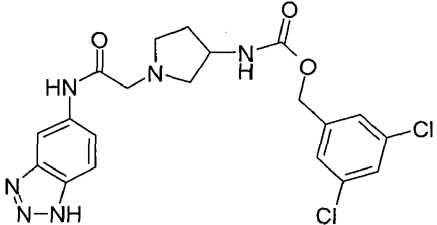 |
| | {1-[(1H-benzotriazol-5-ylcarbamoyl)méthyl]pyrrolidin-3-yl}-carbamate de 3,5-dichlorobenzyle |
| "A17" | 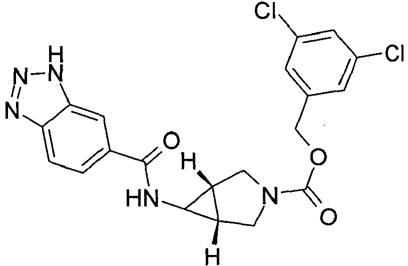 |
| | (1R,5S)-6-[(3H-benzotriazole-5-carbonyl)amino]-3-azabicyclo[3.1.0]hexane-3-carboxylate de 3,5-dichlorobenzyle |
| "A18" | 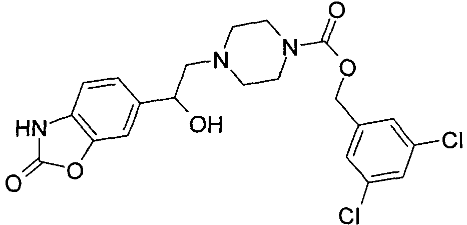 |
| | 4-[2-hydroxy-2-(2-oxo-2,3-dihydrobenzoxazol-6-yl)éthyl]-pipérazine-1-carboxylate de 3,5-dichlorobenzyle |
et les sels et stéréoisomères pharmaceutiquement utilisables de ceux-ci, y compris des mélanges de ceux-ci en toutes proportions.

9. Procédé de préparation de composés de formule I et de sels et stéréoisomères pharmaceutiquement utilisables de ceux-ci, **caractérisé en ce que**
a) pour la préparation de composés de formule I dans laquelle
R désigne 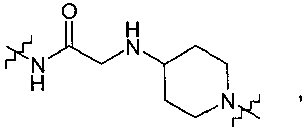 un composé de formule II
Hét-NH-CO-CH₂-L II
dans laquelle
Hét revêt la signification indiquée selon la revendication 1,
et L désigne Cl ou Br,
est réagi avec un composé de formule III 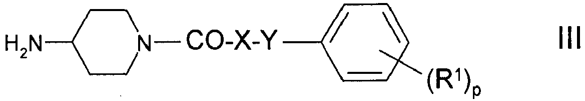 dans laquelle
X, Y, R¹ et p revêtent les significations indiquées selon la revendication 1,
ou
b) pour la préparation de composés de formule I dans laquelle
R désigne 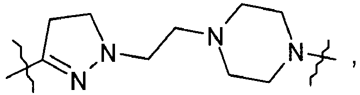 un composé de formule IV 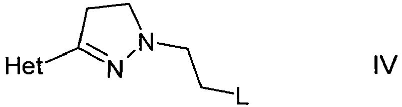 dans laquelle
Hét revêt la signification indiquée selon la revendication 1,
et L désigne Cl ou Br,
est réagi avec un composé de formule V 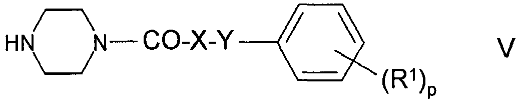 dans laquelle
X, Y, R¹ et p revêtent les significations indiquées selon la revendication 1,
ou
c) pour la préparation de composés de formule I dans laquelle
R désigne 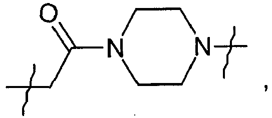 un composé de formule VI
Hét-CH₂-CO-L VI
dans laquelle
Hét revêt la signification indiquée selon la revendication 1,
et L désigne Cl, Br, I ou un groupement OH libre ou modifié de manière réactive et fonctionnelle,
est réagi avec un composé de formule V,
ou
d) pour la préparation de composés de formule I dans laquelle
R désigne 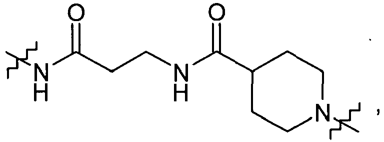 un composé de formule VII 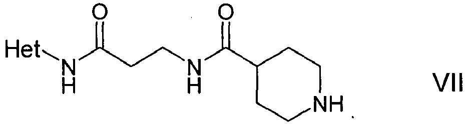 dans laquelle
Hét revêt la signification indiquée selon la revendication 1,
est réagi avec un composé de formule VIII 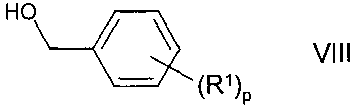 dans laquelle
R¹ et p revêtent les significations indiquées selon la revendication 1,
et un composé choisi dans le groupe constitué par le carbonyldiimidazole, le phosgène, le diphosgène, le triphosgène,
ou
e) pour la préparation de composés de formule I dans laquelle
R désigne 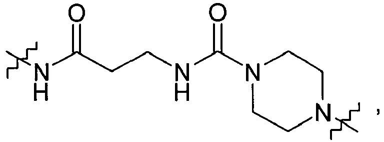 un composé de formule IX 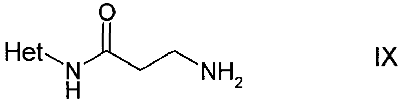 dans laquelle
Hét revêt la signification indiquée selon la revendication 1,
est réagi avec un composé de formule V
et un composé choisi dans le groupe constitué par le carbonyldiimidazole, le phosgène, le diphosgène, le triphosgène,
ou
f) pour la préparation de composés de formule I dans laquelle
R désigne 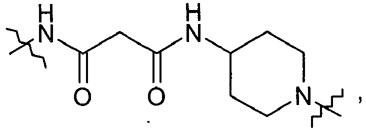 un composé de formule X
Hét-NH₂ X
dans laquelle
Hét revêt la signification indiquée selon la revendication 1,
est réagi avec un composé de formule XI 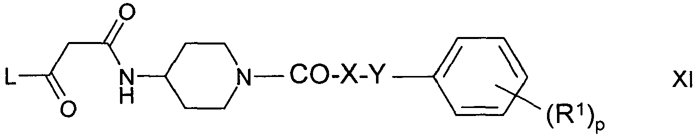 dans laquelle
X, Y, R¹, p revêtent les significations indiquées selon la revendication 1,
et L désigne Cl, Br, I ou un groupement OH libre ou modifié de manière réactive et fonctionnelle,
et/ou une base ou un acide de formule I est converti(e) en l'un de ses sels.

10. Médicaments comprenant au moins un composé de formule I selon les revendications 1 à 8 et/ou des sels et stéréoisomères pharmaceutiquement utilisables de celui-ci, y compris des mélanges de ceux-ci en toutes proportions, et éventuellement des excipients et/ou des adjuvants.

11. Utilisation de composés selon les revendications 1 à 8, pour la préparation d'un médicament destiné au traitement et à la prophylaxie de maladies cancéreuses.

12. Utilisation selon la revendication 11, dans laquelle les maladies cancéreuses sont associées à une tumeur issue du groupe des tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

13. Utilisation selon la revendication 12, dans laquelle la tumeur est issue du groupe de la leucémie monocytique, de l'adénocarcinome du poumon, des carcinomes du poumon à petites cellules, du cancer du pancréas, du carcinome de l'ovaire, des glioblastomes et du carcinome du sein et du carcinome du côlon.

14. Utilisation selon la revendication 13, dans laquelle la maladie à traiter est une tumeur du sang et du système immunitaire.

15. Utilisation selon la revendication 14, dans laquelle la tumeur est issue du groupe de la leucémie myéloïde aiguë, la leucémie myéloïde chronique, la leucémie lymphatique aiguë et/ou la leucémie lymphatique chronique.

16. Utilisation de composés de formule I selon les revendications 1 à 8 et/ou de sels et solvats physiologiquement acceptables de ceux-ci, pour la préparation d'un médicament destiné au traitement de tumeurs, dans laquelle une quantité thérapeutiquement efficace d'un composé de formule I est administrée en association avec une radiothérapie et un composé issu du groupe : 1) un modulateur du récepteur des oestrogènes, 2) un modulateur du récepteur des androgènes, 3) un modulateur du récepteur des rétinoïdes, 4) un agent cyto-toxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

17. Composé de 6-(2-{4-[3-(4-chlorophénoxy)propionyl]pipérazin-1-yl}-acétyl)-3H-benzoxazol-2-one, et les sels et stéréoisomères pharmaceutiquement utilisables de celui-ci, y compris des mélanges de ceux-ci en toutes proportions.
